# EUROPEAN PATENT APPLICATION

(11) **EP 4 471 024 A1**
(43) Date of publication of application: **04.12.2024**
(21) Application number: 23746452.4
(22) Date of filing: 29.01.2023
(51) Int. Cl.: C07D 405/06, A61K 31/4178, C07C 63/08, A61P 27/08, A61P 27/06, A61P 27/12

(54) **M-CHOLINE RECEPTOR AGONIST COMPOUND, PREPARATION METHOD THEREFOR AND USE THEREOF**

(30) Priority: 29.01.2022 CN 202210113094; 11.07.2022 CN 202210809410
(71) Applicant: Nanjing Gritpharma Co., Ltd., Nanjing, Jiangsu 211112 (CN)
(72) Inventor: CHEN, Lei, Nanjing, Jiangsu 211112 (CN); TIAN, Chao, Nanjing, Jiangsu 211112 (CN); WANG, Nana, Nanjing, Jiangsu 211112 (CN); XIONG, Ruju, Nanjing, Jiangsu 211112 (CN); PENG, Shenzhen, Nanjing, Jiangsu 211112 (CN); QI, Shi, Nanjing, Jiangsu 211112 (CN); ZHU, Min, Nanjing, Jiangsu 211112 (CN); PENG, Yixing, Nanjing, Jiangsu 211112 (CN); CAI, Tongle, Nanjing, Jiangsu 211112 (CN); LU, Yingyan, Nanjing, Jiangsu 211112 (CN); WU, Xiaotao, Nanjing, Jiangsu 211112 (CN); LI, Zhan, Nanjing, Jiangsu 211112 (CN)
(74) Representative: Gille Hrabal Partnerschaftsgesellschaft mbB Patentanwälte
(86) International application number: PCT/CN2023/073718
(87) International publication number: WO 2023/143575

(57) **Abstract**

The present invention relates to an M-choline receptor agonist compound as shown in formula (A), and to a preparation method therefor and a use thereof. The compound shown in formula (A) of the present invention has a significant effect when used for eye diseases, particularly presbyopia, or when used for miosis, and is fast-acting, long-lasting and high-strength.

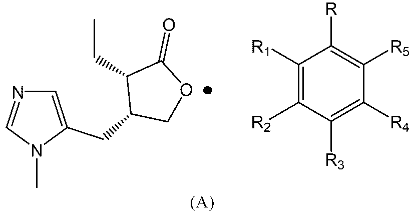

## Description

The present application claims priority to the prior application with the patent application No. 202210113094.8 and entitled "M-CHOLINE RECEPTOR AGONIST COMPOUND, PREPARATION METHOD THEREFOR AND USE THEREOF" filed with China National Intellectual Property Administration on January 29, 2022, and the prior application with the patent application No. 202210809710.5 entitled "CHOLINE ESTER PHENYLACETATE DERIVATIVE, PREPARATION METHOD THEREFOR AND USE THEREOF" filed with China National Intellectual Property Administration on July 11, 2022, the contents of which are incorporated herein by reference in their entirety.

### TECHNICAL FIELD

The present disclosure relates to the technical field of pharmaceutical compounds and particularly relates to an M-choline receptor agonist compound, a preparation method therefor, and use thereof.

### BACKGROUND

Presbyopia is a common vision problem that occurs in middle-aged and elderly individuals. As age increases, the lens of the eye gradually hardens and thickens, and the accommodative ability of the eye muscles also diminishes. This leads to a gradual loss of accommodative function, resulting in a decrease in the zoom ability. When looking at nearby objects, the image is blurred due to the inability to fully focus when projected onto the retina. Presbyopia commonly occurs after the age of 40 and can significantly affect people's quality of life and productivity if left uncorrected. (Frick et al., 2015, Ophthalmology, 122(8): 1706-1710; Goertz et al., 2014, Acta Ophthalmologica, 92(6): 497-500; Patel et al., 2007, Community eye health/International Centre for Eye Health, 20(63): 40-41).

The main symptom of presbyopia is gradually blurred vision when performing close-up tasks (reading, sewing, working in front of a computer, and the like). Presbyopia is one of the diseases with relatively high global morbidity, significantly impacting the quality of life of middle-aged and elderly individuals. With the greatly extended screen time on devices such as smartphones, the incidence of presbyopia is rising yearly.

The primary treatment for presbyopia is the traditional and typical way of wearing presbyopic glasses, but long-term wearing of presbyopic glasses can easily lead to eye fatigue, headaches, dizziness, and other symptoms. Additionally, the presbyopic glasses need to be worn and taken off frequently and are inconvenient to carry.

Currently, there are some candidate drugs for alleviating or treating presbyopia that are in the development stage. For example, Liquid vision (PRX-100), which contains a low dose of aceclidine, is an anticholinesterase drug that can increase the depth of focus by constricting the pupil, thereby allowing patients to achieve near vision. CSF-1 drop (Orasis) is a drug that acts on the parasympathetic nervous system, in which a non-steroidal anti-inflammatory drug is incorporated in an oily base. It can cause pupil constriction and increase the eye's depth of focus, thereby ameliorating presbyopia symptoms. In addition, some topical formulations are also in different stages of development.

There are also drug-device combination products used for presbyopia that are in clinical research. Arctic Vision announced that its ARVN003 has entered Phase III clinical trials. This is an ophthalmic spray of pilocarpine hydrochloride based on a micro-dosing device. The developer asserts that this device can avoid spillage during eye drop application, thereby reducing the risk of drug or preservative exposure due to overmedication. However, this drug-device combination also has some commercialization drawbacks. Besides concerns about its price, its administration concentration is relatively high.

In November 2021, the U.S. Food and Drug Administration (FDA) approved the ophthalmic drug Vuity (pilocarpine HCl, 1.25% eye drops) from Allergan (a subsidiary of Abbvie) for the treatment of presbyopia. This is the world's first topical eye drop for presbyopia. As an M-choline receptor agonist, it is dropped once daily in both eyes, achieving an onset time of about 15 min and a duration of effect of about 4-6 h. Patients receiving Vuity treatment experienced improvements in near and intermediate vision, while their distance vision remained unaffected.

However, Vuity, as the first drug for presbyopia, still has some drawbacks. For instance, 5 min after drop application, some patients experience symptoms such as bloodshot eyes, ocular redness and swelling, and ocular discomfort, and there are also adverse events such as strong eye irritation and headaches (with an incidence rate of over 5%). Meanwhile, the onset time, duration of effect, and comfort of use of Vuity (pilocarpine HCl) all need to be improved.

### SUMMARY

To solve the technical problems described above, the present disclosure provides a compound represented by formula (A), a pharmaceutically acceptable hydrate or solvate thereof: 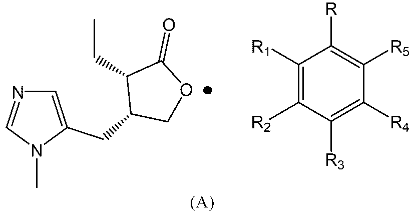
wherein R₁, R₂, R₃, R₄, and R₅ are identical or different, and are each independently selected from hydrogen, C₁₋₆ alkyl, halogen, cyano, and -NR'R", wherein R' and R" are identical or different, and are each independently selected from H, C₁₋₆ alkyl, C₃₋₁₀ cycloalkyl, C₆₋₁₄ aryl, 5- to 14-membered heteroaryl, and 3- to 10-membered heterocyclyl;
R is selected from COOH or 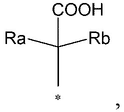 wherein Ra and Rb are identical or different, and are each independently selected from hydrogen, C₁₋₆ alkyl, halogen, cyano, C₃₋₁₀ cycloalkyl, C₆₋₁₄ aryl, 5- to 14-membered heteroaryl, and 3- to 10-membered heterocyclyl;
the C₁₋₆ alkyl, C₃₋₁₀ cycloalkyl, C₆₋₁₄ aryl, 5- to 14-membered heteroaryl and 3- to 10-membered heterocyclyl are optionally substituted with one, two or more of the following groups: oxo, thio, halogen, cyano, -NR'R", -COOH, hydroxyl, C₁₋₆ alkyl, C₁₋₆ alkoxy, C₁₋₆ alkylthio, C₃₋₁₀ cycloalkyl, C₆₋₁₄ aryl, and 5- to 14-membered heteroaryl.

According to an embodiment of the present disclosure, the compound represented by formula (A) is selected from a compound represented by formula I-1 or formula 1-2 shown below:

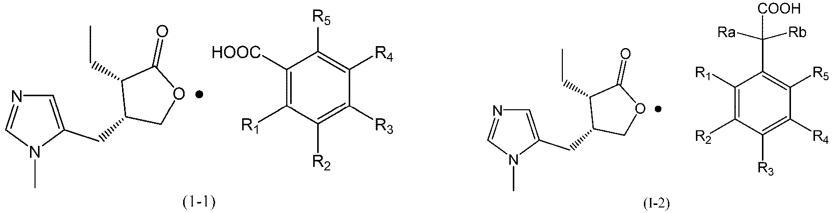

In some embodiments, in formula I-1, R₁, R₂, R₃, R₄ and R₅ are identical or different, and are each independently selected from hydrogen, C₁₋₆ alkyl, halogen, cyano, and amino.

In some embodiments, in formula I-1, R₁, R₂, R₃, R₄ and R₅ are identical or different, and are each independently selected from hydrogen, methyl, ethyl, isopropyl, chlorine, fluorine, and cyano.

In some embodiments, in formula I-1, at least three of R₁, R₂, R₃, R₄ and R₅ are H.

In some embodiments, in formula I-1, four of R₁, R₂, R₃, R₄ and R₅ are H.

In some embodiments, in formula I-1, R₁, R₂, R₄, and R₅ are all H, and R₃ is C₁₋₆ alkyl, halogen, cyano, or -NR'R".

In some embodiments, in formula I-1, R₁, R₂, R₃, and R₅ are all H, and R₄ is C₁₋₆ alkyl, halogen, cyano, or -NR'R".

In some embodiments, in formula I-2, R₁, R₂, R₃, R₄ and R₅ are identical or different, and are each independently selected from hydrogen, C₁₋₆ alkyl, halogen, -COOR', wherein R' is selected from H and C₁₋₆ alkyl.

In some embodiments, in formula I-2, R₁, R₂, R₃, R₄ and R₅ are identical or different, and are each independently selected from hydrogen, methyl, ethyl, isopropyl, chlorine, fluorine, and bromine.

In some embodiments, in formula I-2, Ra and Rb are identical or different, and are each independently selected from hydrogen, C₁₋₆ alkyl, halogen, and cyano, preferably hydrogen or C₁₋₆ alkyl.

In some embodiments, in formula I-2, R₁, R₂, R₃, R₄ and R₅ are identical or different, at least two of which are hydrogen, and preferably three or four of which are hydrogen.

In some embodiments, the compound represented by formula I-1 is selected from the following:

| **Compound** | **Structure** |
|---|---|
| JQ-1 | 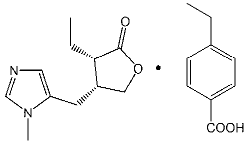 |
| JQ-2 | 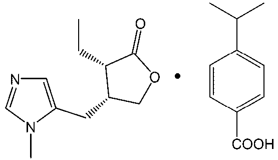 |
| JQ-3 | 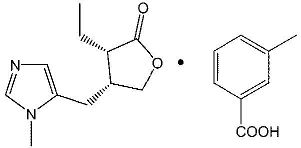 |
| JQ-5 | 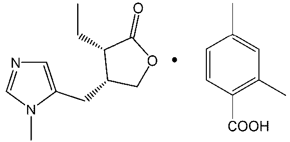 |
| JQ-6 | 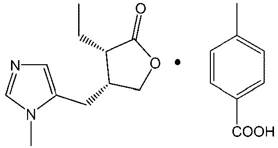 |
| JQ-8 | 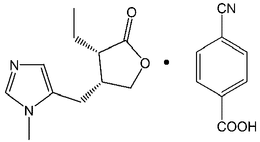 |
| JQ-9 | 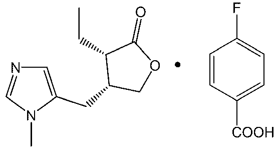 |
| JQ-10 | 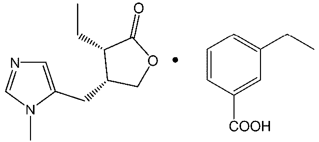 |

The compound represented by formula I-2 is selected from the following:

| No. | Compound |
|---|---|
| 1 | 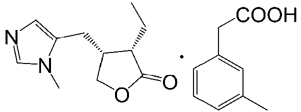 |
| 2 | 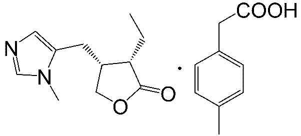 |
| 3 | 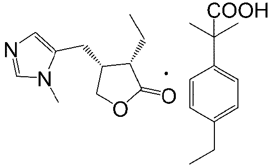 |
| 4 | 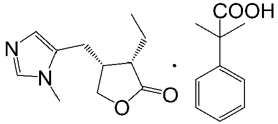 |
| 5 | 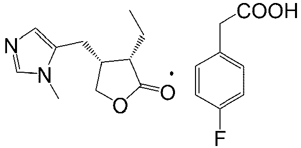 |
| 6 | 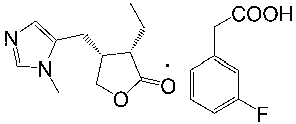 |
| 7 | 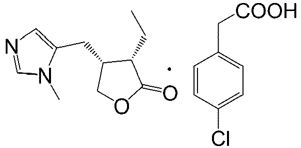 |
| 8 | 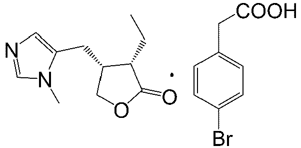 |
| 9 | 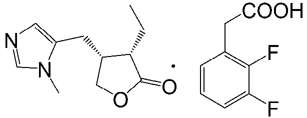 |
| 10 | 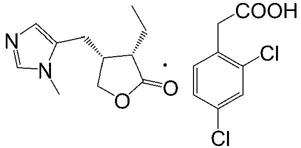 |
| 11 | 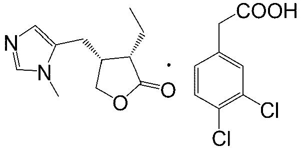 |
| 12 | 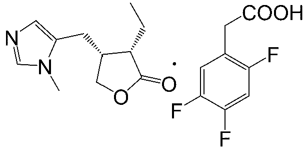 |
| 13 | 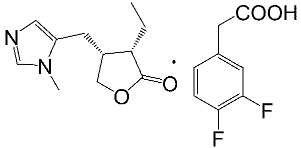 |
| 14 | 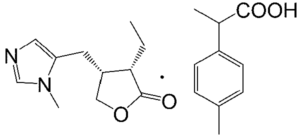 |
| 15 | 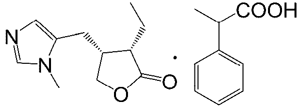 |

Preferably, the compound of formula I-1 is:

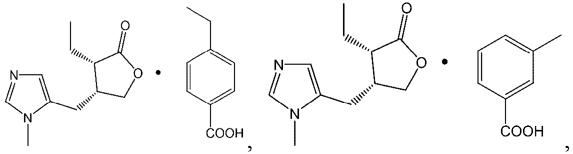

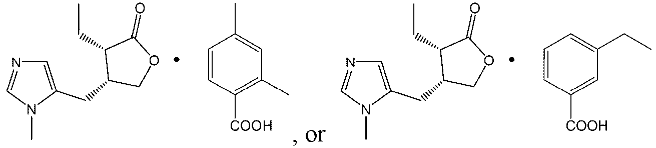

Preferably, the compound of formula I-2 is selected from:

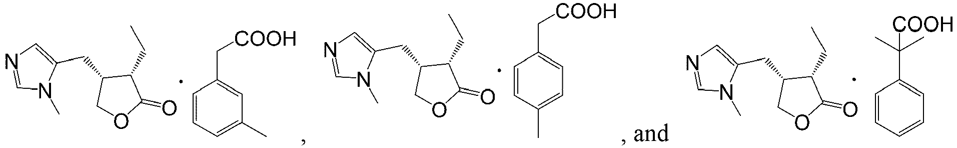

The present disclosure further provides a preparation method for the compound represented by formula (A) as described above, comprising: reacting pilocarpine with a compound of formula (A-1) to give the compound represented by formula (A): 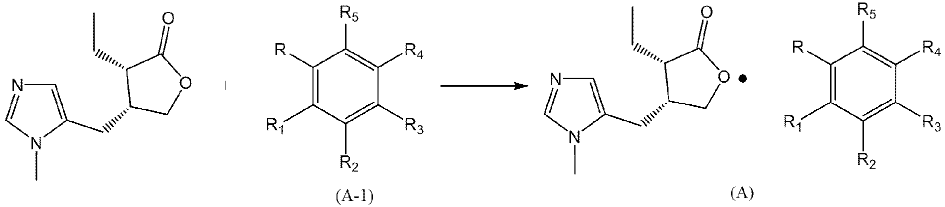 wherein R, R₁, R₂, R₃, R₄, and R₅ are as defined above.

According to an embodiment of the present disclosure, the molar ratio of pilocarpine to the compound of formula (A-1) is 1:1.

According to an embodiment of the present disclosure, the reaction is performed at room temperature.

The present disclosure further provides a pharmaceutical composition, which comprises at least one of the compound represented by formula (A) and the pharmaceutically acceptable hydrate and solvate thereof as described above.

According to an embodiment of the present disclosure, the pharmaceutical composition is used for the prevention or treatment of an ocular disease or disorder, wherein the ocular disease or disorder is at least one of presbyopia, cataract, glaucoma, dry eye syndrome, diabetic retinopathy, macular degeneration, optic neuritis, and retinitis pigmentosa.

According to an embodiment of the present disclosure, in the pharmaceutical composition, the mass concentration of the compound represented by formula (A) is 0.3%-8%, preferably 0.3%-4% or 0.88%-3.5%.

According to an embodiment of the present disclosure, the pharmaceutical composition further comprises a pharmaceutically acceptable carrier or excipient, such as a humectant, a transdermal absorption enhancer, a preservative, an embedding agent, a film-forming agent, or the like.

According to an embodiment of the present disclosure, the pharmaceutical composition is formulated for oral administration, parenteral administration, ocular administration, topical administration, injection, subcutaneous administration, transdermal administration, rectal administration, buccal administration, or transmucosal administration.

According to a preferred embodiment of the present disclosure, the pharmaceutical composition is formulated for ocular administration.

According to an embodiment of the present disclosure, the pharmaceutical composition can be prepared in the following dosage forms: a suspension, a gel, an ointment, an injectable solution, a spray, or an eye drop formulation.

According to an embodiment of the present disclosure, the pharmaceutical composition can be formulated into a compound formulation with other drugs, for example, by adding an antiallergic drug, an ingredient with a synergistic effect, an ingredient for improving the comfort level, and the like.

According to a preferred embodiment of the present disclosure, the pharmaceutical composition is in the form of an eye drop formulation.

The present disclosure further provides the use of the compound represented by formula (A) for the manufacturing of a medicament for the amelioration or treatment of an ocular disease or disorder. According to an embodiment of the present disclosure, the ocular disease or disorder is at least one of presbyopia, cataract, glaucoma, dry eye syndrome, diabetic retinopathy, macular degeneration, optic neuritis, and retinitis pigmentosa.

According to a preferred embodiment of the present disclosure, the ocular disease or disorder is presbyopia.

The present disclosure further provides use of at least one of the compounds represented by formula (A) and the pharmaceutically acceptable hydrate and solvate thereof for the manufacturing of a medicament for miosis.

According to an embodiment of the present disclosure, the therapeutically effective amount of the compound is 0.001-1000 mg per recipient.

### Beneficial Effects

The compound of the present disclosure has a significant effect when used for ocular diseases, especially presbyopia, or when used for ocular miosis, with rapid onset, long duration of effect, and high strength of the effect.

Specifically, for pilocarpine benzoic acid series compounds represented by formula I-1:
The researchers of the present disclosure unexpectedly found that in the screening of various salt forms of pilocarpine, the pilocarpine benzoic acid series compounds overall exhibit a superior pupil constriction effect compared to pilocarpine hydrochloride. At comparable test doses, it is shown that: 1) the pilocarpine benzoic acid series compounds of the present disclosure exhibited a superior initial pupil constriction effect at 5 min compared to the pilocarpine hydrochloride, achieving a faster onset of effect; 2) the measured value of a pilocarpine hydrochloride solution at the time point of 3 h was -3.13 ± 7.35, indicating that the solution had substantially no pupil constriction effect; however, the series compounds of the present application still exhibited a significant pupil constriction effect when measured at the time point of 3 h, and the effect lasted for 4 h or even longer, extending the duration of effect by no less than 33% compared to the pilocarpine hydrochloride; 3) the maximum percentage change in pupil diameter caused by using the pilocarpine hydrochloride was only around 35%, whereas the maximum percentage change in pupil diameter caused by using the series compounds of the present application could reach 40%-45%, with the maximum strength of effect enhanced by 15%-30%. It can be seen that the pilocarpine benzoic acid series compounds of the present disclosure exhibit a faster onset of effect, longer duration of effect, and greater strength of effect, with extremely significant differences in various technical indicators. The efficacy of the pilocarpine benzoic acid series compounds of the present disclosure can achieve the same or even stronger effect compared to the pilocarpine hydrochloride at half the dose of the pilocarpine hydrochloride.

Furthermore, as can be seen from the comparison results of the irritation to rabbit eyes caused by the pilocarpine hydrochloride solution and pilocarpine benzoic acid series solutions, the irritation to the rabbit eyes caused by the pilocarpine benzoic acid series compounds of the present disclosure is equivalent to that caused by the pilocarpine hydrochloride. If the dose is further reduced, adverse effects can be significantly reduced while achieving the same therapeutic effect.

Specifically, for the pilocarpine phenylacetate series compounds represented by formula I-2:
Tests show that compared to other pilocarpine salt series, the duration of the miosis effect of the pilocarpine phenylacetate series is extended by at least 1 h, and the duration of efficacy is relatively extended by no less than 33%, thereby increasing the duration of efficacy and reducing the frequency of administration.

Pilocarpine phenylacetate series solutions cause less irritation to the eyes and exhibit a faster onset of effect, with extremely significant differences in various technical indicators. In addition, the pilocarpine phenylacetate series compound solutions still achieve the same effect as the pilocarpine hydrochloride at half of the molar concentration of the pilocarpine hydrochloride, thereby allowing for a reduced administration dose.

The active pharmaceutical ingredient and formulation of the pilocarpine phenylacetate series overall exhibit superior stability compared to those of the pilocarpine hydrochloride series, with a slow increasing rate of related impurities and easy quality control. Furthermore, the salt-forming ratio in the pilocarpine phenylacetate series solutions can be maintained around 1:1 under different pH conditions, remaining stable for a long time without precipitation.

### Definitions and Description

"Oxo" refers to "=O".

"Halogen" refers to fluorine, chlorine, bromine, or iodine. Preferably, the halogen is fluorine, chlorine, or bromine.

"C₁₋₆ alkyl" should be understood to represent a linear or branched saturated monovalent hydrocarbyl group having 1-6 carbon atoms, such as methyl, ethyl, n-propyl, n-butyl, isopropyl, isobutyl, sec-butyl, or tert-butyl. More particularly, the group has 1, 2, or 3 carbon atoms ("C₁₋₃ alkyl"), such as methyl, ethyl, n-propyl, or isopropyl.

"C₁₋₆ alkoxy" refers to the group -O-C₁₋₆ alkyl, wherein the C₁₋₆ alkyl is defined as above.

"C₁₋₆ alkylthio" should be understood as -S-C₁₋₆ alkyl, wherein the C₁₋₆ alkyl is defined as above. "C₃₋₁₀ cycloalkyl" should be understood to represent a saturated monovalent monocyclic or bicyclic hydrocarbon ring having 3, 4, 5, 6, 7, 8, 9, or 10 carbon atoms. The C₃₋₁₀ cycloalkyl can be a monocyclic hydrocarbyl group such as cyclopropyl, cyclobutyl, cyclopentyl, cyclohexyl, cycloheptyl, cyclooctyl, cyclononyl, or cyclodecyl, or can be a bicyclic hydrocarbyl group such as a decahydronaphthalene ring.

"C₆₋₁₄ aryl" should be understood to represent an aromatic or partially aromatic monovalent monocyclic, bicyclic or tricyclic hydrocarbon ring having 6, 7, 8, 9, 10, 11, 12, 13, or 14 carbon atoms ("C₆₋₁₄ aryl"), in particular a ring having 6 carbon atoms ("C₆ aryl"), such as phenyl or biphenyl; a ring having 9 carbon atoms ("C₉ aryl"), such as indanyl or indenyl; a ring having 10 carbon atoms ("C₁₀ aryl"), such as tetrahydronaphthyl, dihydronaphthyl or naphthyl; a ring having 13 carbon atoms ("C₁₃ aryl"), such as fluorenyl; or a ring having 14 carbon atoms ("C₁₄ aryl"), such as anthryl.

"5- to 14-membered heteroaryl" should be understood to represent a monovalent monocyclic, bicyclic or tricyclic aromatic ring system that has 5, 6, 7, 8, 9, 10, 11, 12, 13 or 14 ring atoms, in particular 5, 6, 9 or 10 carbon atoms, contains 1-5, preferably 1-3 heteroatoms independently selected from N, O and S, and may be benzo-fused in each case. In particular, the heteroaryl is selected from thienyl, furanyl, pyrrolyl, oxazolyl, thiazolyl, imidazolyl, pyrazolyl, isoxazolyl, isothiazolyl, oxadiazolyl, triazolyl, thiadiazolyl, thia-4H-pyrazolyl and the like and benzo derivatives thereof, such as benzofuranyl, benzothienyl, benzoxazolyl, benzoisoxazolyl, benzimidazolyl, benzotriazolyl, indazolyl, indolyl, isoindolyl and the like; or pyridinyl, pyridazinyl, pyrimidinyl, pyrazinyl, triazinyl and the like and benzo derivatives thereof, such as quinolyl, quinazolinyl, isoquinolyl and the like; or azocinyl, indolizinyl, purinyl and the like and benzo derivatives thereof; or cinnolinyl, phthalazinyl, quinazolinyl, quinoxalinyl, naphthyridinyl, pteridinyl, carbazolyl, acridinyl, phenazinyl, phenothiazinyl, phenoxazinyl, and the like.

"3- to 10-membered heterocyclyl" refers to a saturated monovalent monocyclic or bicyclic hydrocarbon ring containing 1-5, preferably 1-3 heteroatoms selected from N, O, and S. The heterocyclyl may be connected to the rest of the molecule through any one of the carbon atoms or the nitrogen atom(s) (if present). In particular, the heterocyclyl may include, but is not limited to: 4-membered rings such as azetidinyl and oxetanyl; 5-membered rings such as tetrahydrofuranyl, dioxolyl, pyrrolidinyl, imidazolidinyl, pyrazolidinyl and pyrrolinyl; 6-membered rings such as tetrahydropyranyl, piperidyl, morpholinyl, dithianyl, thiomorpholinyl, piperazinyl and trithianyl; or 7-membered rings such as diazepanyl. Optionally, the heterocyclyl may be benzo-fused. The heterocyclyl may be bicyclic, for example, but not limited to, a 5,5-membered ring such as a hexahydrocyclopenta[*c*]pyrrol-2(1*H*)-yl ring, or a 5,6-membered bicyclic ring such as a hexahydropyrrolo[1,2-*a*]pyrazin-2(1*H*)-yl ring. The ring containing the nitrogen atom may be partially unsaturated, i.e., it may contain one or more double bonds, for example, but not limited to, 2,5-dihydro-1*H*-pyrrolyl, 4*H*-[1,3,4]thiadiazinyl, 4,5-dihydrooxazolyl, or 4*H*-[1,4]thiazinyl, or it may be benzo-fused, for example, but not limited to, dihydroisoquinolyl.

"Pharmaceutically acceptable carrier" refers to a carrier that can be used in the preparation of a pharmaceutical composition, which is generally compatible with the other ingredients in the composition and harmless to the recipient.

"Pharmaceutically acceptable carrier" includes one or more than one carrier, and embodiments thereof include carriers for topical, ocular, parenteral, intravenous, intraperitoneal, intramuscular, sublingual, nasal and oral administration. "Pharmaceutically acceptable carrier" also includes reagents used in the preparation of an aqueous dispersion and sterile powders for injection or dispersion.

As used herein, "excipient" includes a physiologically compatible additive that can be used in the preparation of a pharmaceutical composition. Examples of the pharmaceutically acceptable carrier and excipient can be found, for example, in Remington Pharmaceutical Science (16th edition).

"Therapeutically effective amount" refers to a dose of a compound or composition that is effective in affecting the amelioration of presbyopia or for use in ocular miosis and other related ocular disorders. As used herein, the term may also refer to an effective amount to produce a desired *in-vivo* effect (amelioration of presbyopia or miosis) in an animal, preferably a human.

### BRIEF DESCRIPTION OF THE DRAWINGS

FIG. 1 shows the images of changes in irritation to rabbit eyes caused by 1.25% pilocarpine hydrochloride;
FIG. 2 shows the images of changes in irritation to rabbit eyes caused by a JQ-1 solution at a concentration of 1.82%;
FIG. 3 shows the images of changes in irritation to rabbit eyes caused by a JQ-2 solution at a concentration of 1.90%;
FIG. 4 shows the images of changes in irritation to rabbit eyes caused by a JQ-3 solution at a concentration of 1.75%;
FIG. 5 shows the images of changes in irritation to rabbit eyes caused by a JQ-5 solution at a concentration of 0.91%;
FIG. 6 shows the images of changes in irritation to rabbit eyes caused by a JQ-6 solution at a concentration of 1.75%;
FIG. 7 shows the images of changes in irritation to rabbit eyes caused by a JQ-8 solution at a concentration of 1.81%;
FIG. 8 shows the images of changes in irritation to rabbit eyes caused by a JQ-9 solution at a concentration of 1.77%;
FIG. 9 shows the images of changes in irritation to rabbit eyes caused by a JQ-10 solution at a concentration of 1.82%;
FIG. 10 shows the images of irritation to rabbit eyes in normal saline, hydrochloride, and nitrate groups in Test Example 3;
FIG. 11 shows the images of irritation to rabbit eyes in salicylate and malate groups in Test Example 3;
FIG. 12 shows the images of irritation to rabbit eyes in maleate and fumarate groups in Test Example 3;
FIG. 13 shows the images of irritation to rabbit eyes in a 0.88% JQ-3 p-toluate group in Test Example 4;
FIG. 14 shows the images of irritation to rabbit eyes in a 3.50% JQ-3 p-toluate group in Test Example 4;
FIG. 15 shows the images of irritation to rabbit eyes caused by compound 1 at a concentration of 1.83% in Test Example 1;
FIG. 16 shows the images of irritation to rabbit eyes caused by compound 2 at a concentration of 1.83% in Test Example 1; and
FIG. 17 shows the images of irritation to rabbit eyes caused by compound 4 at a concentration of 1.90% in Test Example 1.

### DETAILED DESCRIPTION

The technical solutions of the present disclosure will be further described in detail with reference to the following specific examples. It should be understood that the following examples are merely exemplary illustrations and explanations of the present disclosure and should not be construed as limiting the protection scope of the present disclosure. All techniques implemented based on the contents described above in the present disclosure are encompassed within the protection scope of the present disclosure.

Unless otherwise stated, the starting materials and reagents used in the following examples are all commercially available products or can be prepared by using known methods.

The concentrations of eye drop solutions involved in the following examples refer to mass concentrations.

The following specifically describes the preparation of compounds JQ-1 to JQ-10.

### Example 1. Preparation of Pilocarpine

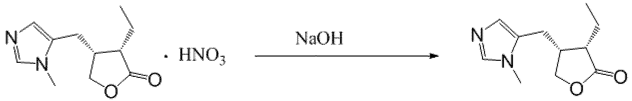

At room temperature, pilocarpine nitrate (15.00 g, 55.30 mmol, 1.0 eq) was dissolved in H₂O (30 mL), and a 1 mol/L aqueous NaOH solution (58.00 mL, 58.07 mmol, 1.05 eq) was added dropwise. The mixture was stirred at room temperature for 30 min. TLC analysis showed the completion of the reaction. 50 mL of dichloromethane was added to the reaction solution for extraction, and this procedure was repeated three times. The organic phases were combined, washed with H₂O (30 mL × 1) and a saturated aqueous NaCl solution (30 mL × 1), dried over anhydrous Na₂SO₄, and filtered. The filtrate was collected and dried under reduced pressure to give 12.23 g of a colorless liquid, i.e., 12.23 g of pilocarpine (yield: 79.36%).

### Example 2. Preparation of Compound JQ-1

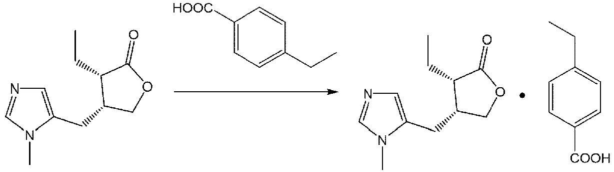

At room temperature, pilocarpine (2.00 g, 9.60 mmol, 1.0 eq) was dissolved in EtOH (10 mL), and 4-ethylbenzoic acid (1.44 g, 9.60 mmol, 1.0 eq) was added. The mixture was stirred at room temperature for 1 h. TLC showed the completion of the reaction. The solvent was removed by rotary evaporation. The residue was left at -20 °C overnight to give a white solid, and the resulting solid was slurried with 20 mL of n-hexane for 1 h and filtered. The filter cake was collected and dried under reduced pressure to give 3.31 g of a white solid (yield: 96.21%).

¹H-NMR characterization: ¹H NMR (400 MHz, DMSO- *d₆*) δ 7.87 (d, J = 8.1 Hz, 2H), 7.56 (s, 1H), 7.33 (d, J = 8.1 Hz, 2H), 6.74 (s, 1H), 4.21 (dd, J = 8.9, 5.8 Hz, 1H), 3.97 (dd, J = 9.0, 3.1 Hz, 1H), 3.55 (s, 3H), 2.90 - 2.80 (m, 1H), 2.77 - 2.62 (m, 4H), 2.36 (dd, J = 15.5, 11.4 Hz, 1H), 1.75 - 1.63 (m, 1H), 1.58 - 1.47 (m, 1H), 1.20 (t, J = 7.6 Hz, 3H), 1.02 (t, J = 7.4 Hz, 3H).

### Example 3. Preparation of Compound JQ-2

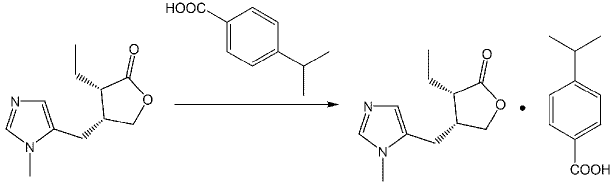

At room temperature, pilocarpine (2.00 g, 9.60 mmol, 1.0 eq) was dissolved in EtOH (10 mL), and 4-isopropylbenzoic acid (1.58 g, 9.60 mmol, 1.0 eq) was added. The mixture was stirred at room temperature for 1 h. TLC showed the completion of the reaction. The solvent was removed by rotary evaporation. The residue was left at -20 °C overnight to give a white solid, and the resulting solid was slurried with 20 mL of n-hexane for 1 h and filtered. The filter cake was collected and dried under reduced pressure to give 3.42 g of a white solid (yield: 95.59%).

¹H-NMR characterization: ¹H NMR (400 MHz, DMSO-*d*₆) *δ* 7.87 (d, *J =* 8.3 Hz, 2H), 7.55 (s, 1H), 7.36 (d, *J =* 8.2 Hz, 2H), 6.74 (s, 1H), 4.21 (dd, *J =* 8.9, 5.8 Hz, 1H), 3.97 (dd, *J =* 9.0, 3.1 Hz, 1H), 3.55 (s, 3H), 2.01 - 2.91 (m, 1H), 2.90 - 2.80 (m, 1H), 2.76- 2.64 (m, 2H), 2.36 (dd, *J =* 15.6, 11.4 Hz, 1H), 1.74 - 1.63 (m, 1H), 1.59 - 1.46 (m, 1H), 1.22 (d, *J =* 6.9 Hz, 3H), 1.02 (t, *J =* 7.4 Hz, 3H).

### Example 4. Preparation of Compound JQ-3

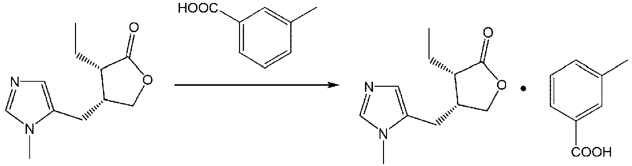

At room temperature, pilocarpine (2.00 g, 9.60 mmol, 1.0 eq) was dissolved in EtOH (10 mL), and m-toluic acid (1.31 g, 9.60 mmol, 1.0 eq) was added. The mixture was stirred at room temperature for 1 h. TLC showed the completion of the reaction. The solvent was removed by rotary evaporation. The residue was left at -20 °C overnight to give a white solid, and the resulting solid was slurried with 20 mL of n-hexane for 1 h and filtered. The filter cake was collected and dried under reduced pressure to give 3.13 g of a white solid (yield: 94.71%).

¹H-NMR characterization: ¹H NMR (400 MHz, DMSO-*d*₆) *δ* 7.88 (d, *J =* 8.8 Hz, 2H), 7.54 (s, 1H), 7.00 (d, *J =* 8.9 Hz, 2H), 6.73 (s, 1H), 4.21 (dd, *J =* 8.8, 5.8 Hz, 1H), 3.98 (m, 3H), 3.55 (s, 3H), 2.91 - 2.80 (m, 1H), 2.70 (m, 2H), 2.36 (dd, *J =* 15.6, 11.4 Hz, 1H), 1.80 - 1.62 (m, 3H), 1.53 (m, 1H), 1.00 (m, 6H).

### Example 5. Preparation of Compound JQ-5

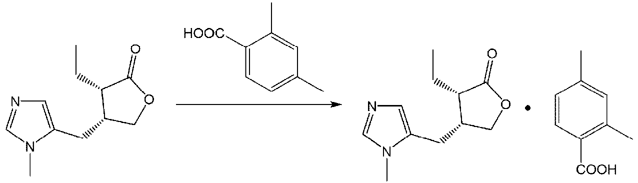

At room temperature, pilocarpine (2.00 g, 9.60 mmol, 1.0 eq) was dissolved in EtOH (10 mL), and 2,4-dimethylbenzoic acid (1.44 g, 9.60 mmol, 1.0 eq) was added. The mixture was stirred at room temperature for 1 h. TLC showed the completion of the reaction. The solvent was removed by rotary evaporation. The residue was left at -20 °C overnight to give a white solid, and the resulting solid was slurried with 20 mL of n-hexane for 1 h and filtered. The filter cake was collected and dried under reduced pressure to give 3.30 g of a white solid (yield: 95.86%).

¹H-NMR characterization: ¹H NMR (400 MHz, DMSO-*d*₆) *δ* 7.74 (d, *J =* 7.8 Hz, 1H), 7.54 (s, 1H), 7.10 (t, J =12Hz, 2H), 6.73 (s, 1H), 4.21 (dd, *J =* 8.9, 5.8 Hz, 1H), 3.97 (dd, *J =* 9.0, 3.1 Hz, 1H), 3.55 (s, 3H), 2.90 - 2.80 (m, 1H), 2.70 (m,2H), 2.49 (s, 3H), 2.36 (dd, *J =* 15.6, 11.5 Hz, 1H), 2.31 (s, 3H), 1.75 - 1.62 (m, 1H), 1.59 - 1.46 (m, 1H), 1.02 (t, *J =* 7.4 Hz, 3H).

### Example 6. Preparation Method for Compound JQ-6

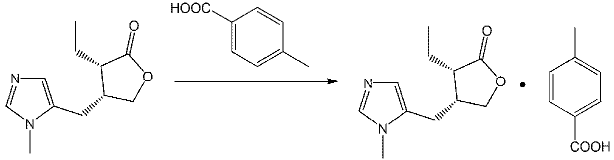

At room temperature, pilocarpine (2.00 g, 9.60 mmol, 1.0 eq) was dissolved in EtOH (10 mL), and p-toluic acid (1.31 g, 9.60 mmol, 1.0 eq) was added. The mixture was stirred at room temperature for 1 h. TLC showed the completion of the reaction. The reaction mixture was then concentrated by rotary evaporation to remove the solvent. The residue was left at -20 °C overnight to give a white solid, and the resulting solid was slurried with 20 mL of n-hexane for 1 h and filtered. The filter cake was collected and dried under reduced pressure to give 3.12 g of a white solid (yield: 94.23%). ¹H-NMR characterization: ¹H NMR (400 MHz, DMSO-*d*₆) *δ* 7.84 (d, *J =* 8.1 Hz, 2H), 7.55 (s, 1H), 7.30 (d, *J =* 7.9 Hz, 2H), 6.74 (s, 1H), 4.21 (dd, *J =* 8.6, 5.8 Hz, 1H), 3.97 (dd, *J =* 9.0, 3.1 Hz, 1H), 3.55 (s, 3H), 2.85 (m, 1H), 2.70 (m, 2H), 2.41 - 2.31 (m, 4H), 1.75 - 1.62 (m, 1H), 1.53 (m, 1H), 1.02 (t, *J* = 7.4 Hz, 2H).

### Example 7. Preparation of Compound JQ-8

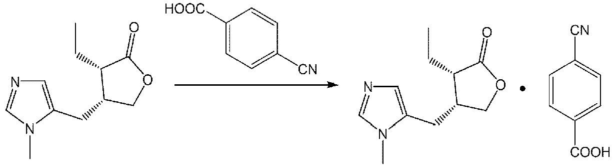

At room temperature, pilocarpine (2.00 g, 9.60 mmol, 1.0 eq) was dissolved in EtOH (10 mL), and p-cyanobenzoic acid (1.41 g, 9.60 mmol, 1.0 eq) was added. The mixture was stirred at room temperature for 1 h. TLC showed the completion of the reaction. The solvent was removed by rotary evaporation. The residue was left at -20 °C overnight to give a white solid, and the resulting solid was slurried with 20 mL of n-hexane for 1 h and filtered. The filter cake was collected and dried under reduced pressure to give 3.23 g of a white solid (yield: 94.69%).

¹H-NMR characterization: ¹H NMR (400 MHz, DMSO-*d*₆) *δ* 8.12 - 8.06 (m, 2H), 8.00 - 7.94 (m, 2H), 7.67 (s, 1H), 6.80 (s, 1H), 4.22 (dd, *J =* 8.8, 5.6 Hz, 1H), 3.97 (dd, *J =* 9.0, 3.2 Hz, 1H), 3.57 (s, 3H), 2.90 - 2.82 (m, 1H), 2.77 - 2.63 (m, 2H), 2.38 (dd, *J =* 15.6, 11.4 Hz, 1H), 1.72 - 1.65 (m, 1H), 1.57 - 1.49 (m, 1H), 1.02 (t, *J=* 7.4 Hz, 3H).

### Example 8. Preparation of Compound JQ-9

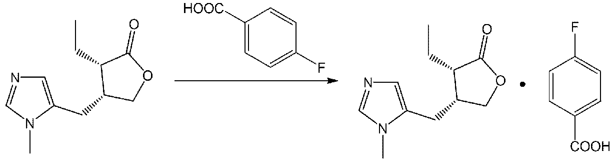

At room temperature, pilocarpine (2.00 g, 9.60 mmol, 1.0 eq) was dissolved in EtOH (10 mL), and p-fluorobenzoic acid (1.35 g, 9.60 mmol, 1.0 eq) was added. The mixture was stirred at room temperature for 1 h. TLC showed the completion of the reaction. The solvent was removed by rotary evaporation. The residue was left at -20 °C overnight to give a white solid, and the resulting solid was slurried with 20 mL of n-hexane for 1 h and filtered. The filter cake was collected and dried under reduced pressure to give 3.17 g of a white solid (yield: 94.67%).

¹H-NMR characterization: ¹H NMR (400 MHz, DMSO-*d*₆) *δ* 8.05 - 7.97 (m, 2H), 7.56 (s, 1H), 7.32 (m, 2H), 6.74 (s, 1H), 4.22 (dd, *J =* 8.9, 5.8 Hz, 1H), 3.97 (dd, *J =* 9.0, 3.1 Hz, 1H), 3.55 (s, 3H), 2.91 - 2.80 (m, 1H), 2.74 - 2.64 (m, 2H), 2.36 (dd, *J =* 15.6, 11.4 Hz, 1H), 1.74 - 1.63 (m, 1H), 1.58 - 1.47 (m, 1H), 1.02 (t, *J =* 7.4 Hz, 3H).

### Example 9. Preparation of Compound JQ-10

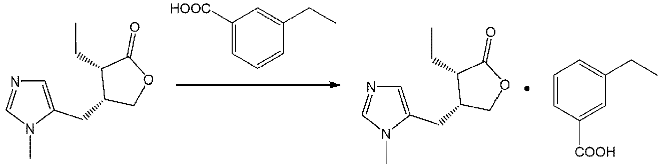

At room temperature, pilocarpine (2.00 g, 9.60 mmol, 1.0 eq) was dissolved in EtOH (10 mL), and 3-ethylbenzoic acid (1.44 g, 9.60 mmol, 1.0 eq) was added. The mixture was stirred at room temperature for 1 h. TLC showed the completion of the reaction. The solvent was removed by rotary evaporation. The residue was left at -20 °C overnight to give a white solid, and the resulting solid was slurried with 20 mL of n-hexane for 1 h and filtered. The filter cake was collected and dried under reduced pressure to give 3.25 g of a white solid (yield: 94.48%).

¹H-NMR characterization: ¹H NMR (400 MHz, DMSO-*d*₆) δ 7.77(t, J = 12.0, 2H), 7.55 (s, 1H), 7.48 - 7.39 (m, 2H), 6.73 (s, 1H), 4.21 (dd, J = 8.8, 5.8 Hz, 1H), 3.96 (dd, J = 9.0, 3.0 Hz, 1H), 3.54 (s, 3H), 2.89 - 2.81 (m, 1H), 2.76- 2.64 (m, 4H), 2.36 (dd, J = 15.5, 11.5 Hz, 1H), 1.74 - 1.63 (m, 1H), 1.58 - 1.47 (m, 1H), 1.20 (t, J = 7.6 Hz, 3H), 1.01 (t, J = 7.4 Hz, 3H).

The following synthesis scheme illustrates the specific synthetic route for the compound of formula (I-2) of the present disclosure:

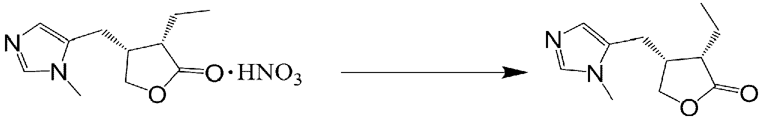

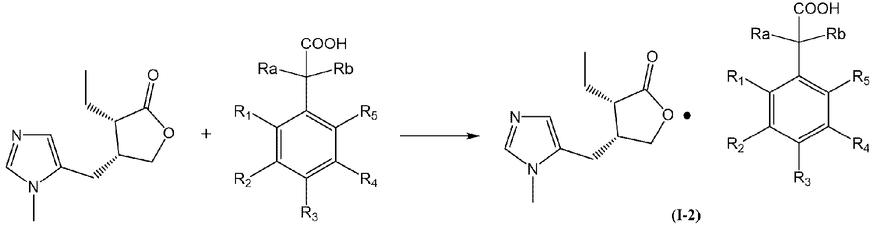

### Example 10

### Preparation of Compound 1

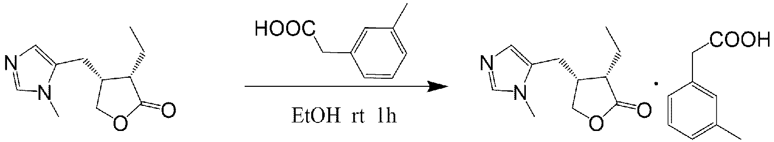

At room temperature, pilocarpine (5.00 g, 24 mmol, 1.0 eq) was dissolved in EtOH (15 mL), and 3-methylphenylacetic acid (3.61 g, 24 mmol, 1.0 eq) was added. The mixture was stirred at room temperature for 1 h. HPLC showed the completion of the reaction. The solvent was removed by rotary evaporation. The residue was left at -20 °C overnight to give a white solid, and the resulting solid was slurried with 16 mL of n-hexane for 1 h and filtered. The filter cake was collected and dried under reduced pressure to give a white solid, i.e., compound 1 (8.42 g, yield: 97.8%).

¹H NMR (400 MHz, DMSO-*d*₆) δ 7.53 (s, 1H), 7.20 (t, J = 7.6 Hz, 1H), 7.09 - 7.01 (m, 3H), 6.73 (s, 1H), 4.21 (dd, J = 8.9, 5.8 Hz, 1H), 3.96 (dd, J = 9.0, 3.1 Hz, 1H), 3.54 (s, 3H), 3.51 (s, 2H), 2.90 - 2.80 (m, 1H), 2.73 (dd, J = 15.4, 7.3 Hz, 1H), 2.66 (dd, J = 15.6, 4.3 Hz, 1H), 2.35 (dd, J = 15.6, 11.4 Hz, 1H), 2.29 (s, 3H), 172 - 1.65 (m, 1H), 1.59 - 1.46 (m, 1H), 1.02 (t, J = 7.4 Hz, 3H).

### Example 11

### Preparation of Compound 2

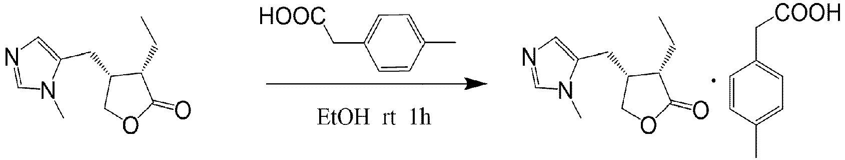

At room temperature, pilocarpine (5.00 g, 24 mmol, 1.0 eq) was dissolved in EtOH (15 mL), and 4-methylphenylacetic acid (3.61 g, 24 mmol, 1.0 eq) was added. The mixture was stirred at room temperature for 1 h. HPLC showed the completion of the reaction. The solvent was removed by rotary evaporation. The residue was left at -20 °C overnight to give a white solid, and the resulting solid was slurried with 16 mL of n-hexane for 1 h and filtered. The filter cake was collected and dried under reduced pressure to give a white solid, i.e., compound 2 (8.40 g, yield: 97.6%).

¹H NMR (400 MHz, DMSO-*d*₆) δ 7.54 (s, 1H), 7.17-7.09 (m, 4H), 6.73 (s, 1H), 4.21 (dd, J = 8.9, 5.8 Hz, 1H), 3.96 (dd, J = 9.0, 3.1 Hz, 1H), 3.54 (s, 3H), 3.50 (s, 2H), 2.90 - 2.80 (m, 1H), 2.73 (dd, J = 15.2, 7.5 Hz, 1H), 2.66 (dd, J = 15.6, 4.3 Hz, 1H), 2.36 (dd, J = 15.6, 11.4 Hz, 1H), 2.28 (s, 3H), 1.75 - 1.62 (m, 1H), 1.59 - 1.46 (m, 1H), 1.02 (t, J = 7.4 Hz, 3H).

### Example 12

### Preparation of Compound 3

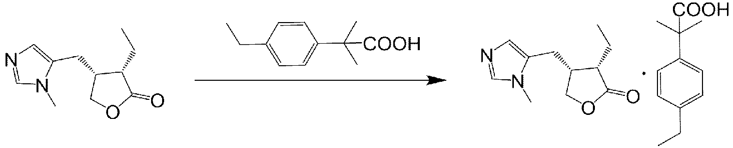

At room temperature, pilocarpine (3.80 g, 18 mmol, 1.0 eq) was dissolved in EtOH (15 mL), and 2-(4-ethylphenyl)-2-methylpropionic acid (3.50 g, 18 mmol, 1.0 eq) was added. The mixture was stirred at room temperature for 1 h. HPLC showed the completion of the reaction. The solvent was removed by rotary evaporation. The residue was left at -20 °C overnight to give a white solid, and the resulting solid was slurried with 12 mL of n-hexane for 1 h and filtered. The filter cake was collected and dried under reduced pressure to give a white solid, i.e., compound 3 (6.95 g, yield: 95.2%). ¹H NMR (400 MHz, DMSO-*d*₆) δ 7.53 (s, 1H), 7.25 (d, *J =* 8.0 Hz, 2H), 7.16 (d, *J =* 8.3 Hz, 2H), 6.72 (s, 1H), 4.21 (dd, *J =* 8.9, 5.7 Hz, 1H), 3.96 (dd, *J =* 9.0, 3.1 Hz, 1H), 3.54 (s, 3H), 2.90 - 2.80 (m, 1H), 2.76 - 2.64 (m, 2H), 2.57 (q, *J =* 7.6 Hz, 2H), 2.35 (dd, *J =* 15.6, 11.4 Hz, 1H), 1.75 - 1.47 (m, 2H), 1.45 (s, 6H), 1.17 (t, *J=* 7.6 Hz, 3H), 1.02 (t, *J =* 7.4 Hz, 3H).

### Example 13

### Preparation of Compound 4

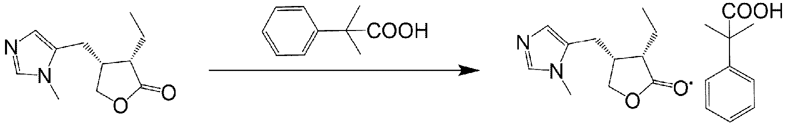

At room temperature, pilocarpine (3.84 g, 18 mmol, 1.0 eq) was dissolved in EtOH (15 mL), and 2-phenylisobutyric acid (3.03 g, 18 mmol, 1.0 eq) was added. The mixture was stirred at room temperature for 1 h. HPLC showed the completion of the reaction. The solvent was removed by rotary evaporation. The residue was left at -20 °C overnight to give a white solid, and the resulting solid was slurried with 12 mL of n-hexane for 1 h and filtered. The filter cake was collected and dried under reduced pressure to give a white solid, i.e., compound 4 (6.60 g, yield: 96.1%). ¹H NMR (400 MHz, DMSO-*d*₆) δ 7.54 (s, 1H), 7.40 - 7.29 (m, 4H), 7.28 - 7.19 (m, 1H), 6.73 (s, 1H), 4.21 (dd, *J =* 8.8, 5.8 Hz, 1H), 3.97 (dd, *J =* 9.0, 3.0 Hz, 1H), 3.54 (s, 3H), 2.89 - 2.81 (m, 1H), 2.76 - 2.64 (m, 2H), 2.36 (dd, *J =* 15.5, 11.4 Hz, 1H), 1.76 - 1.50 (m, 2H), 1.47 (s, 6H), 1.02 (t, *J =* 7.4 Hz, 3H).

### Example 14

### Preparation of Compound 5

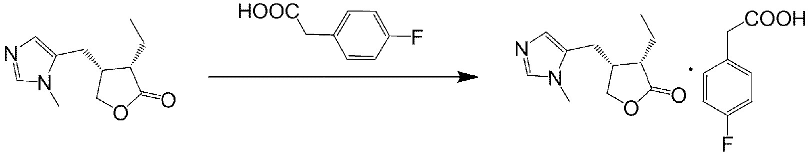

At room temperature, pilocarpine (3.35 g, 16.1 mmol, 1.0 eq) was dissolved in EtOH (15 mL), and 4-fluorophenylacetic acid (2.48 g, 16.1 mmol, 1.0 eq) was added. The mixture was stirred at room temperature for 1 h. HPLC showed the completion of the reaction. The solvent was removed by rotary evaporation. The residue was left at -20 °C overnight to give a white solid, and the resulting solid was slurried with 10 mL of n-hexane for 1 h and filtered. The filter cake was collected and dried under reduced pressure to give a white solid, i.e., compound 5 (5.48 g, yield: 94.0%). ¹H NMR (400 MHz, DMSO-*d*₆) δ 7.58 (s, 1H), 7.30 (dd, J = 8.4, 5.7 Hz, 2H), 7.13 (t, J = 8.9 Hz, 2H), 6.76 (s, 1H), 4.21 (dd, J = 8.9, 5.8 Hz, 1H), 3.97 (dd, J = 9.0, 3.0 Hz, 1H), 3.57 (s, 2H), 3.55 (s, 3H), 2.89 - 2.81 (m, 1H), 2.76 - 2.64 (m, 2H), 2.36 (dd, J = 15.5, 11.5 Hz, 1H), 1.76 - 1.45 (m, 2H), 1.02 (t, J = 7.4 Hz, 3H).

### Example 15

### Preparation of Compound 6

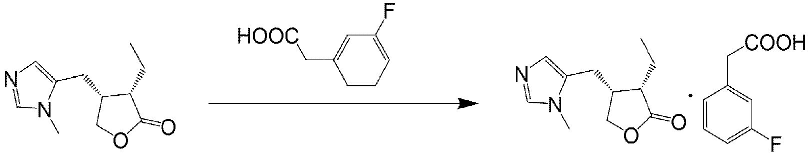

At room temperature, pilocarpine (3.27 g, 15.7 mmol, 1.0 eq) was dissolved in EtOH (15 mL), and 3-fluorophenylacetic acid (2.42 g, 15.7 mmol, 1.0 eq) was added. The mixture was stirred at room temperature for 1 h. HPLC showed the completion of the reaction. The solvent was removed by rotary evaporation. The residue was left at -20 °C overnight to give a white solid, and the resulting solid was slurried with 10 mL of n-hexane for 1 h and filtered. The filter cake was collected and dried under reduced pressure to give a white solid, i.e., compound 6 (5.60 g, yield: 98.4%). ¹H NMR (400 MHz, DMSO-*d*₆) δ 7.56 (s, 1H), 7.35 (dd, *J =* 15.0, 7.3 Hz, 1H), 7.09 (dd, *J =* 16.6, 8.0 Hz, 3H), 6.74 (s, 1H), 4.21 (dd, *J =* 8.8, 5.9 Hz, 1H), 3.97 (dd, *J =* 9.0, 2.9 Hz, 1H), 3.62 (s, 2H), 3.55 (s, 3H), 2.87 - 2.83 (m, 1H), 2.76 - 2.64 (m, 2H), 2.36 (dd, *J=* 15.5, 11.4 Hz, 1H), 1.75 - 1.46 (m, 2H), 1.02 (t, *J =* 7.4 Hz, 3H).

### Example 16

### Preparation of Compound 7

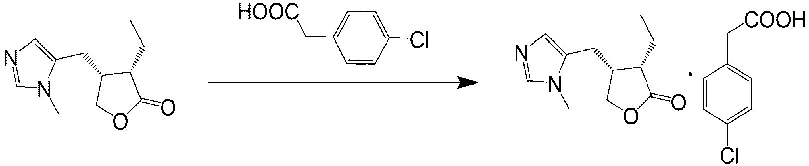

At room temperature, pilocarpine (3.23 g, 15.5 mmol, 1.0 eq) was dissolved in EtOH (15 mL), and 4-chlorophenylacetic acid (2.65 g, 15.5 mmol, 1.0 eq) was added. The mixture was stirred at room temperature for 1 h. HPLC showed the completion of the reaction. The solvent was removed by rotary evaporation. The residue was left at -20 °C overnight to give a white solid, and the resulting solid was slurried with 10 mL of n-hexane for 1 h and filtered. The filter cake was collected and dried under reduced pressure to give a white solid, i.e., compound 7 (5.75 g, yield: 97.8%). ¹H NMR (400 MHz, DMSO-*d*₆) δ 7.55 (s, 1H), 7.37 (d, *J =* 8.4 Hz, 2H), 7.29 (d, *J =* 8.4 Hz, 2H), 6.73 (s, 1H), 4.21 (dd, *J =* 9.0, 5.8 Hz, 1H), 3.97 (dd, *J =* 9.0, 3.1 Hz, 1H), 3.59 (s, 2H), 3.55 (s, 3H), 2.90 - 2.80 (m, 1H), 2.76 - 2.64 (m, 2H), 2.36 (dd, *J =* 15.6, 11.4 Hz, 1H), 1.75 - 1.46 (m, 2H), 1.02 (t, *J* = 7.4 Hz, 3H).

### Example 17

### Preparation of Compound 8

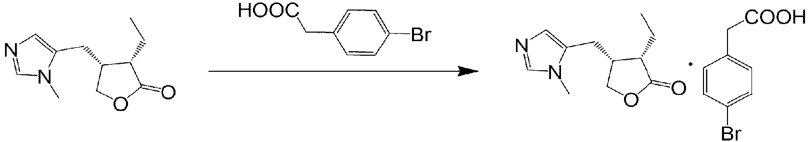

At room temperature, pilocarpine (3.18 g, 15.3 mmol, 1.0 eq) was dissolved in EtOH (15 mL), and 4-bromophenylacetic acid (3.28 g, 15.3 mmol, 1.0 eq) was added. The mixture was stirred at room temperature for 1 h. HPLC showed the completion of the reaction. The solvent was removed by rotary evaporation. The residue was left at -20 °C overnight to give a white solid, and the resulting solid was slurried with 10 mL of n-hexane for 1 h and filtered. The filter cake was collected and dried under reduced pressure to give a white solid, i.e., compound 8 (6.18 g, yield: 95.7%). ¹H NMR (400 MHz, DMSO-*d*₆) δ 7.57 (s, 1H), 7.53 - 7.46 (m, 2H), 7.23 (d, *J =* 8.4 Hz, 2H), 6.75 (s, 1H), 4.21 (dd, *J =* 8.9, 5.7 Hz, 1H), 3.97 (dd, *J =* 9.0, 3.1 Hz, 1H), 3.57 (s, 2H), 3.55 (s, 3H), 2.91 - 2.80 (m, 1H), 2.76 - 2.64 (m, 2H), 2.36 (dd, *J =* 15.6, 11.4 Hz, 1H), 1.75 - 1.46 (m, 2H), 1.02 (t, *J* = 7.4 Hz, 3H).

### Example 18

### Preparation of Compound 9

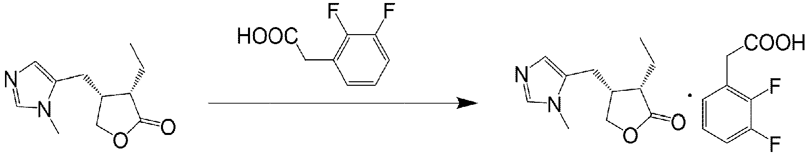

At room temperature, pilocarpine (3.58 g, 17.2 mmol, 1.0 eq) was dissolved in EtOH (15 mL), and 2,3-difluorophenylacetic acid (2.95 g, 17.2 mmol, 1.0 eq) was added. The mixture was stirred at room temperature for 1 h. HPLC showed the completion of the reaction. The solvent was removed by rotary evaporation. The residue was left at -20 °C overnight to give a white solid, and the resulting solid was slurried with 10 mL of n-hexane for 1 h and filtered. The filter cake was collected and dried under reduced pressure to give a white solid, i.e., compound 9 (6.34 g, yield: 97.1%). ¹H NMR (400 MHz, DMSO-*d*₆) δ 7.55 (s, 1H), 7.39 - 7.28 (m, 1H), 7.21 - 7.10 (m, 2H), 6.74 (s, 1H), 2.90 - 2.80 (m, 1H), 2.76 - 2.64 (m, 2H), 2.36 (dd, *J =* 15.6, 11.4 Hz, 1H), 1.75 - 1.46 (m, 2H), 1.02 (t, *J =* 7.4 Hz, 3H).

### Example 19

### Preparation of Compound 10

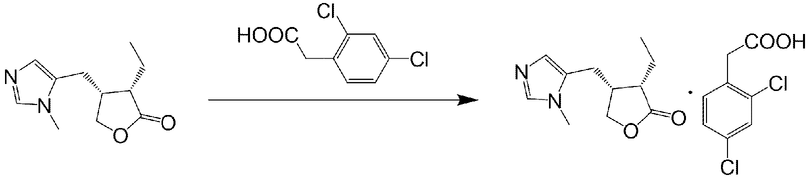

At room temperature, pilocarpine (3.37 g, 16.2 mmol, 1.0 eq) was dissolved in EtOH (15 mL), and 2,4-dichlorophenylacetic acid (3.32 g, 16.2 mmol, 1.0 eq) was added. The mixture was stirred at room temperature for 1 h. HPLC showed the completion of the reaction. The solvent was removed by rotary evaporation. The residue was left at -20 °C overnight to give a white solid, and the resulting solid was slurried with 10 mL of n-hexane for 1 h and filtered. The filter cake was collected and dried under reduced pressure to give a white solid, i.e., compound 10 (6.45 g, yield: 96.4%). ¹H NMR (400 MHz, DMSO-*d*₆) δ 7.61 (d, *J =* 2.0 Hz, 1H), 7.56 (s, 1H), 7.45 - 7.38 (m, 2H), 6.74 (s, 1H), 4.21 (dd, *J =* 8.9, 5.7 Hz, 1H), 3.97 (dd, *J =* 9.0, 3.1 Hz, 1H), 3.72 (s, 2H), 3.55 (s, 3H), 2.91 - 2.79 (m, 1H), 2.76 - 2.64 (m, 2H), 2.36 (dd, *J =* 15.6, 11.4 Hz, 1H), 1.75 - 1.46 (m, 2H), 1.02 (t, *J* = 7.4 Hz, 3H).

### Example 20

### Preparation of Compound 11

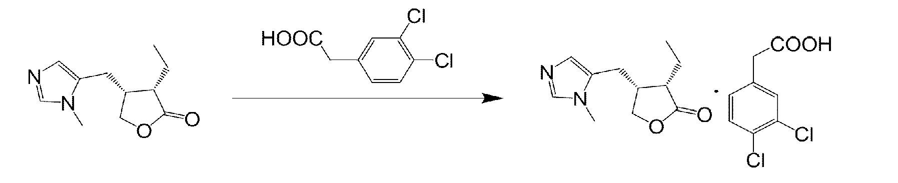

At room temperature, pilocarpine (3.00 g, 14.4 mmol, 1.0 eq) was dissolved in EtOH (15 mL), and 3,4-dichlorophenylacetic acid (2.95 g, 14.4 mmol, 1.0 eq) was added. The mixture was stirred at room temperature for 1 h. HPLC showed the completion of the reaction. The solvent was removed by rotary evaporation. The residue was left at -20 °C overnight to give a white solid, and the resulting solid was slurried with 10 mL of n-hexane for 1 h and filtered. The filter cake was collected and dried under reduced pressure to give a white solid, i.e., compound 11 (5.65 g, yield: 95.0%). ¹H NMR (400 MHz, DMSO-*d*₆) δ 7.59 - 7.55 (m, 3H), 7.27 (dd, *J =* 8.2, 2.0 Hz, 1H), 6.73 (s, 1H), 4.21 (dd, *J =* 9.0, 5.8 Hz, 1H), 3.96 (dd, *J =* 9.0, 3.1 Hz, 1H), 3.64 (s, 2H), 3.55 (s, 3H), 2.90 - 2.80 (m, 1H), 2.76 - 2.64 (m, 2H), 2.36 (dd, *J =* 15.6, 11.4 Hz, 1H), 1.76 - 1.46 (m, 2H), 1.02 (t, *J* = 7.4 Hz, 3H).

### Example 21

### Preparation of Compound 12

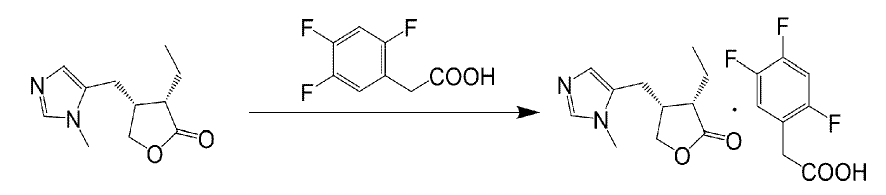

At room temperature, pilocarpine (3.41 g, 16.4 mmol, 1.0 eq) was dissolved in EtOH (15 mL), and 2,4,5-trifluorophenylacetic acid (3.11 g, 16.4 mmol, 1.0 eq) was added. The mixture was stirred at room temperature for 1 h. HPLC showed the completion of the reaction. The solvent was removed by rotary evaporation. The residue was left at -20 °C overnight to give a white solid, and the resulting solid was slurried with 10 mL of n-hexane for 1 h and filtered. The filter cake was collected and dried under reduced pressure to give a white solid, i.e., compound 12 (6.21 g, yield: 95.2%). ¹H NMR (400 MHz, DMSO-*d*₆) δ 7.56 (s, 1H), 7.54-7.44 (m, 2H), 6.74 (s, 1H), 4.21 (dd, J = 8.9, 5.8 Hz, 1H), 3.96 (dd, J = 9.0, 3.1 Hz, 1H), 3.63 (s, 2H), 3.55 (s, 3H), 2.92 - 2.79 (m, 1H), 2.76 - 2.64(m, 2H), 2.36 (dd, J = 15.6, 11.4 Hz, 1H), 1.75 - 1.45 (m, 2H), 1.02 (t, J = 7.4 Hz, 3H).

### Example 22

### Synthesis Method for Compound 13

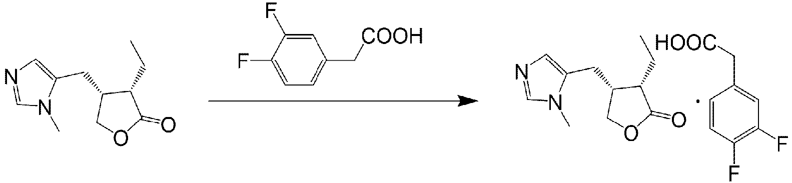

At room temperature, pilocarpine (3.04 g, 15 mmol, 1.0 eq) was dissolved in EtOH (15 mL), and 3,4-difluorophenylacetic acid (2.50 g, 15 mmol, 1.0 eq) was added. The mixture was stirred at room temperature for 1 h. HPLC showed the completion of the reaction. The solvent was removed by rotary evaporation. The residue was left at -20 °C overnight to give a white solid, and the resulting solid was slurried with 12 mL of n-hexane for 1 h and filtered. The filter cake was collected and dried under reduced pressure to give a white solid, i.e., compound 13 (5.34 g, yield: 96.4%).

¹H NMR (400 MHz, DMSO-*d*₆) δ 7.59 (s, 1H), 7.35 (dd, J = 19.5, 8.5 Hz, 2H), 7.14 - 7.07 (m, 1H), 6.76 (s, 1H), 4.21 (dd, J = 8.9, 5.8 Hz, 1H), 3.97 (dd, J = 9.0, 3.0 Hz, 1H), 3.60 (s, 2H), 3.56 (s, 3H), 2.90 - 2.82 (m, 1H), 2.79 - 2.61 (m, 2H), 2.37 (dd, J = 15.6, 11.5 Hz, 1H), 1.76 - 1.46 (m, 2H), 1.02 (t, J = 7.4 Hz, 3H).

### Example 23

### Synthesis Method for Compound 14

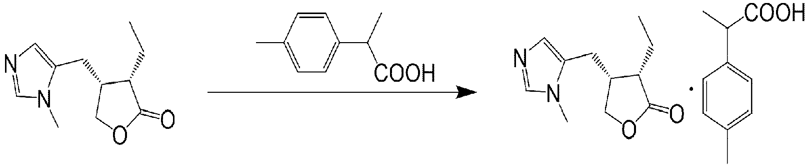

At room temperature, pilocarpine (3.80 g, 18 mmol, 1.0 eq) was dissolved in EtOH (15 mL), and 2-(p-tolyl)propionic acid (3.00 g, 18 mmol, 1.0 eq) was added. The mixture was stirred at room temperature for 1 h. HPLC showed the completion of the reaction. The solvent was removed by rotary evaporation. The residue was left at -20 °C overnight to give a white solid, and the resulting solid was slurried with 12 mL of n-hexane for 1 h and filtered. The filter cake was collected and dried under reduced pressure to give a white solid, i.e., compound 14 (6.55 g, yield: 96.3%).

¹H NMR (400 MHz, DMSO-*d*₆) δ 7.58 (d, J = 6.0 Hz, 1H), 7.35 - 7.27 (m, 4H), 7.26 - 7.20 (m, 1H), 6.75 (d, J = 3.6 Hz, 1H), 4.20 (dd, J = 8.9, 5.8 Hz, 1H), 3.96 (dd, J = 9.0, 2.9 Hz, 1H), 3.67 (q, J = 7.1 Hz, 1H), 3.54 (s, 3H), 2.90 - 2.80 (m, 1H), 2.77 - 2.62 (m, 2H), 2.36 (dd, J = 15.4, 11.6 Hz, 1H), 1.76 - 1.45 (m, 2H), 1.36 (d, J = 7.1 Hz, 3H), 1.01 (t, J = 7.4 Hz, 3H).

### Example 24

### Synthesis Method for Compound 15

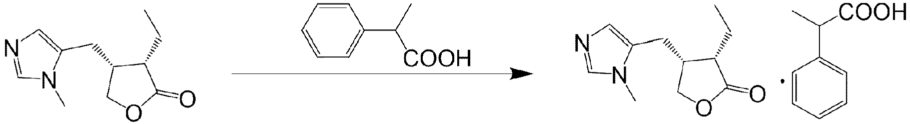

At room temperature, pilocarpine (3.85 g, 18 mmol, 1.0 eq) was dissolved in EtOH (15 mL), 2-phenylpropionic acid (2.78 g, 18 mmol, 1.0 eq) was added. The mixture was stirred at room temperature for 1 h. HPLC showed the completion of the reaction. The solvent was removed by rotary evaporation. The residue was left at -20 °C overnight to give a white solid, and the resulting solid was slurried with 12 mL of n-hexane for 1 h and filtered. The filter cake was collected and dried under reduced pressure to give a white solid, i.e., compound 15 (6.32 g, yield: 95.3%).

¹H NMR (400 MHz, DMSO-*d*₆) δ 7.58 (d, J = 6.0 Hz, 1H), 7.35 - 7.27 (m, 4H), 7.26 - 7.20 (m, 1H), 6.75 (d, J = 3.6 Hz, 1H), 4.20 (dd, J = 8.9, 5.8 Hz, 1H), 3.96 (dd, J = 9.0, 2.9 Hz, 1H), 3.67 (q, J = 7.1 Hz, 1H), 3.54 (s, 3H), 2.90 - 2.80 (m, 1H), 2.77 - 2.62 (m, 2H), 2.36 (dd, J = 15.4, 11.6 Hz, 1H), 1.76 - 1.45 (m, 2H), 1.36 (d, J = 7.1 Hz, 3H), 1.01 (t, J = 7.4 Hz, 3H).

### Test Example 1

### Preparation of Eye Drop Solution:

Reagents: pilocarpine hydrochloride (source: Nanjing Jiqun Pharmaceutical Technology Co., Ltd.), compounds JQ-1, JQ-2, JQ-3, JQ-5, JQ-6, JQ-8, JQ-9, JQ-10, compound 1, compound 2, compound 4 (prepared in the examples described above), and normal saline (specification: 500 mL:4.5 g).

The following eye drop solutions of groups 1) to 12) were separately prepared using normal saline:
1) a pilocarpine hydrochloride solution at a concentration of 1.25% prepared using normal saline;
2) a JQ-1 solution at a concentration of 1.82% prepared using normal saline;
3) a JQ-2 solution at a concentration of 1.90% prepared using normal saline;
4) a JQ-3 solution at a concentration of 1.75% prepared using normal saline;
5) a JQ-5 solution at a concentration of 0.91% prepared using normal saline;
6) a JQ-6 solution at a concentration of 1.75% prepared using normal saline;
7) a JQ-8 solution at a concentration of 1.81% prepared using normal saline;
8) a JQ-9 solution at a concentration of 1.77% prepared using normal saline;
9) a JQ-10 solution at a concentration of 1.82% prepared using normal saline;
10) a compound 1 solution at a concentration of 1.83% prepared using normal saline;
11) a compound 2 solution at a concentration of 1.83% prepared using normal saline; and
12) a compound 4 solution at a concentration of 1.90% prepared using normal saline.

Except for group 5), the eye drop solutions of groups 1) to 12) described above contained an equimolar concentration of pilocarpine. The molar concentration of pilocarpine in the eye drop solution of group 5) was half of the molar concentration of pilocarpine in the solutions of the other 11 groups.

The effects of the eye drop solutions of groups 1) to 12) prepared above on rabbit pupil constriction and irritation were tested separately.

### 1. Materials and methods

### 1.1. Experimental animals

Species and strain: New Zealand rabbit
Grade: normal
Source: Qinglongshan Animal Breeding Farm, Jiangning District, Nanjing
Production license No.: SCXK (Su) 2016-0008
Animal age: about 110 days old at the time of administration
Animal body weight: about 2.5-3.5 kg at the time of administration
Animal sex: all male
Feed: rabbit pellet feed
Housing conditions: air-conditioned room, with a temperature of 18-26 °C and a relative humidity of 40%-80%.

### 1.2. Instrument

Digital vernier caliper: specification: 0-150 mm; measurement accuracy: 0.01 mm; manufacturer: Guilin Guanglu Measuring Instrument Co., Ltd.

### 1.3. Test

### (1) Route of administration

Each rabbit was subjected to eye drop administration in the left eye (using a pipette), with an administration volume of 100 µL.

### (2) Test arrangement

Number of animals: for JQ series compounds, 6 rabbits per compound; for compounds 1, 2 and 4, 5 rabbits per compound.
Administration schedule: single administration.

Measurement indicators and time points:
for JQ series compounds, the pupil diameter of the left eye of the rabbits was measured at time points of -0.5 h (i.e., half an hour before administration), 5 min, 10 min, 15 min, 0.5 h, 3 h and 4 h relative to administration, with the measurement repeated 3 times for each rabbit at each time point (measurement time points were extended if necessary);
for compounds 1, 2 and 4, the pupil diameter of the left eye of the rabbits was measured at time points of 3 h and 4 h relative to administration, with the measurement repeated 3 times for each rabbit at each time point (measurement time points were extended if necessary).
Irritation: the changes in irritation to rabbit eyes were examined concurrently with the pupil diameter at each time point.
Percentage change in pupil diameter (%) after administration = (pupil diameter after administration - pupil diameter before administration)/pupil diameter before administration × 100%

The measurement result for each rabbit at each time point was represented by the average value of three measurements.

### 2.1. Test results

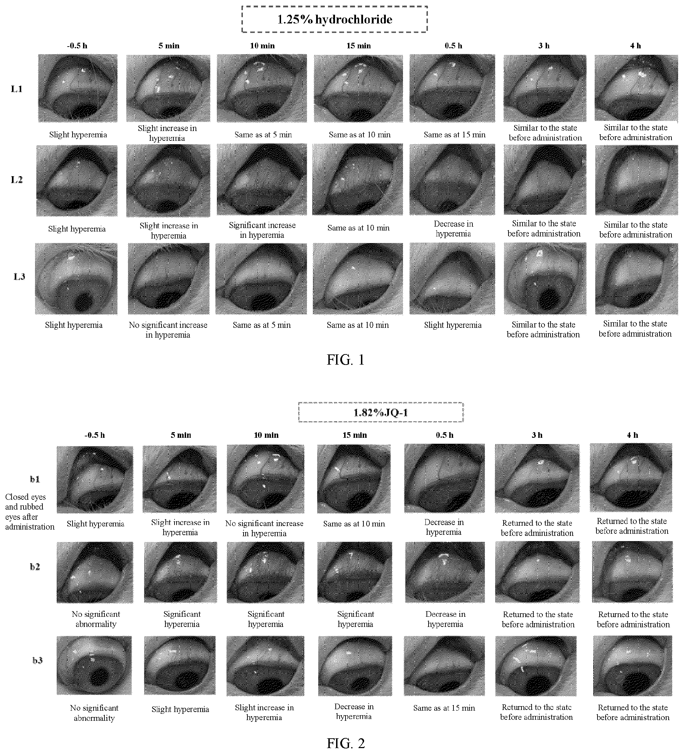

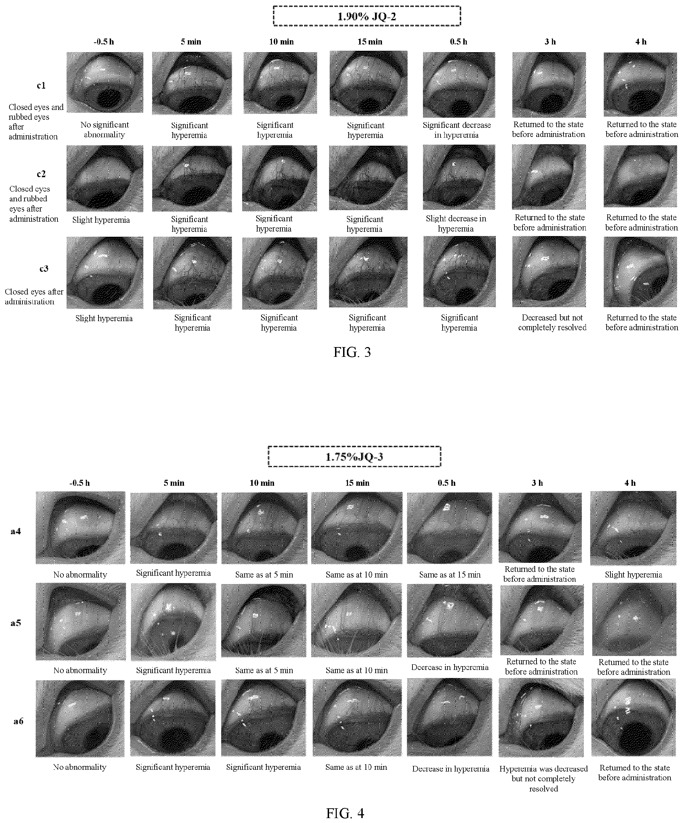

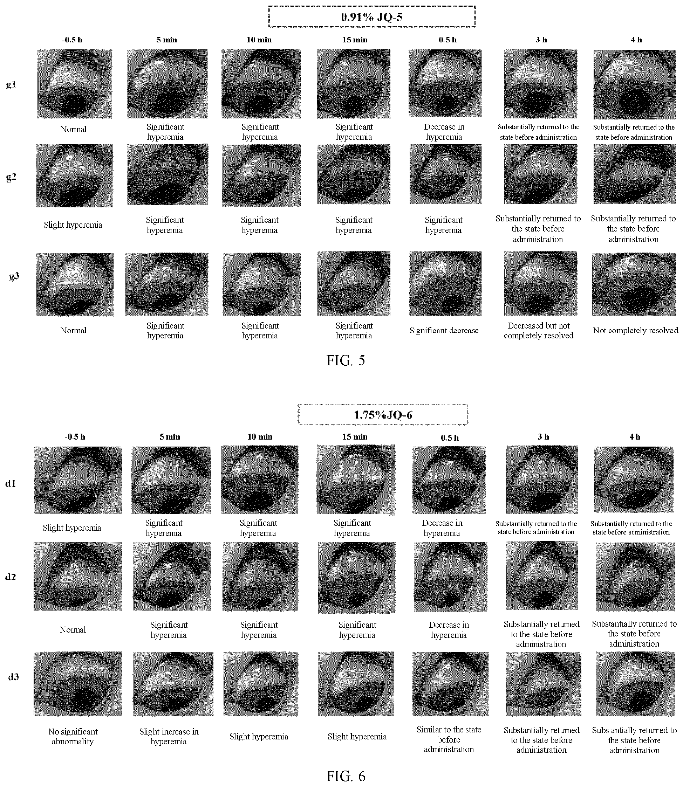

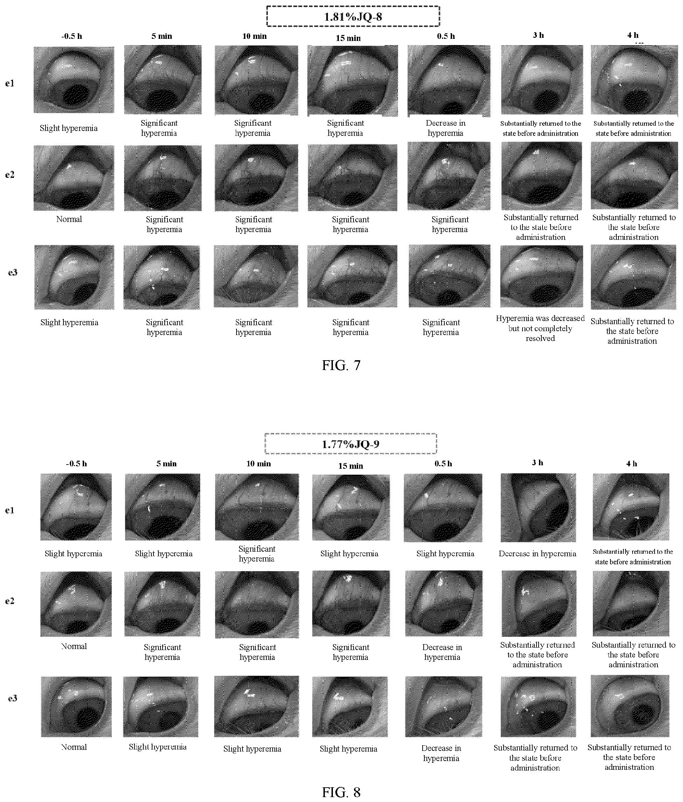

The results of the irritation to rabbit eyes caused by different eye drop solutions of benzoic acid and hydrochloride over different periods are shown in FIGs. 1-9. The test results of rabbit pupil change values are shown in Tables 1-1 to 1-9, and the average value and the standard deviation (x̅ ± SD value) of the percentage changes in pupil diameter (%) at each time point in Tables 1-1 to 1-9 are listed in Table 1-10.

**Table 1-1: Percentage changes in pupil diameter before and after administration of 1.25% pilocarpine hydrochloride solution in six rabbits**

| No. | Percentage change (%) | | | | | | |
|---|---|---|---|---|---|---|---|
| | -0.5 h | 5 min | 10 min | 15 min | 0.5 h | 3 h | 4h |
| L1 | - | -6.51 | -26.64 | -33.71 | -32.89 | -6.71 | 2.49 |
| L2 | - | -15.75 | -36.43 | -42.10 | -39.82 | -16.22 | -2.39 |
| L3 | - | -2.17 | -21.69 | -24.95 | -23.64 | 0.16 | 0.54 |
| L4 | - | -6.28 | -29.40 | -40.68 | -36.45 | 4.54 | -0.15 |
| L5 | - | -7.23 | -32.93 | -36.99 | -36.62 | -0.58 | -1.79 |
| L6 | - | -8.93 | -28.98 | -33.54 | -38.14 | 0.03 | 2.79 |
| Average value | - | -7.81 | -29.35 | -35.33 | -34.59 | -3.13 | 0.25 |
| SD | - | 4.48 | 5.08 | 6.18 | 5.84 | 7.35 | 2.14 |

| | | | | | | | |
|---|---|---|---|---|---|---|---|
| Note: SD means standard deviation. | | | | | | | |

**Table 1-2: Percentage changes in pupil diameter before and after administration of 1.82% JQ-1 solution in six rabbits**

| No. | Percentage change (%) | | | | | | |
|---|---|---|---|---|---|---|---|
| | -0.5 h | 5 min | 10 min | 15 min | 0.5 h | 3 h | 4h |
| b1 | - | -18.30 | -39.48 | -40.98 | -36.94 | -14.51 | -0.15 |
| b2 | - | -12.49 | -43.89 | -48.60 | -43.99 | -17.36 | -0.37 |
| b3 | - | -12.96 | -28.44 | -34.57 | -34.03 | 0.43 | -0.11 |
| b4 | - | -16.32 | -47.06 | -47.55 | -38.42 | -14.17 | -1.05 |
| b5 | - | -30.42 | -43.80 | -47.01 | -45.18 | -29.45 | -14.91 |
| b6 | - | -14.67 | -50.72 | -48.06 | -44.43 | -28.43 | -22.24 |
| Average value | - | -17.53 | -42.23 | -44.46 | -40.50 | -17.25 | -6.47 |
| SD | - | 6.67 | 7.72 | 5.59 | 4.65 | 10.99 | 9.66 |

**Table 1-3: Percentage changes in pupil diameter before and after administration of 1.90% JQ-2 solution in six rabbits**

| **No.** | **Percentage change (%)** | | | | | | |
|---|---|---|---|---|---|---|---|
| | **-0.5 h** | **5 min** | **10 min** | **15 min** | **0.5 h** | **3 h** | **4 h** |
| c1 | - | -10.30 | -40.18 | -45.97 | -42.73 | -11.78 | -1.06 |
| c2 | - | -19.54 | -43.03 | -44.50 | -40.84 | -12.71 | 1.17 |
| c3 | - | -18.81 | -38.38 | -42.71 | -38.73 | -19.42 | 0.10 |
| c4 | - | -11.79 | -44.30 | -40.41 | -39.97 | -14.53 | -0.99 |
| c5 | - | -8.19 | -47.13 | -50.13 | -47.53 | -2.08 | 0.05 |
| c6 | - | -8.62 | -29.99 | -34.69 | -30.62 | -13.90 | 1.72 |
| Average value | - | -12.87 | -40.50 | -43.07 | -40.07 | -12.40 | 0.17 |
| SD | - | 5.05 | 6.00 | 5.25 | 5.56 | 5.71 | 1.12 |

**Table 1-4: Percentage changes in pupil diameter before and after administration of 1.75% JQ-3 solution in six rabbits**

| **No.** | **Percentage change (%)** | | | | | | |
|---|---|---|---|---|---|---|---|
| | **-0.5 h** | **5 min** | **10 min** | **15 min** | **0.5 h** | **3 h** | **4 h** |
| a1 | - | -9.20 | -33.98 | -43.27 | -41.20 | -13.85 | -0.74 |
| a2 | - | -11.21 | -48.26 | -46.77 | -43.07 | -13.69 | 0.33 |
| a3 | - | -8.26 | -34.05 | -39.56 | -36.81 | -2.00 | 10.90 |
| a4 | - | -14.85 | -45.63 | -49.36 | -45.72 | -17.68 | -0.24 |
| a5 | - | -36.44 | -49.44 | -49.60 | -48.02 | -8.83 | 1.69 |
| a6 | - | -18.72 | -47.94 | -48.37 | -48.27 | -19.95 | -0.43 |
| Average value | - | -16.45 | -43.22 | -46.15 | -43.85 | -12.67 | 1.92 |
| SD | - | 10.53 | 7.24 | 3.98 | 4.42 | 6.47 | 4.48 |

**Table 1-5: Percentage changes in pupil diameter before and after administration of 0.91% JQ-5 solution in six rabbits**

| **No.** | **Percentage change (%)** | | | | | | |
|---|---|---|---|---|---|---|---|
| | **-0.5 h** | **5 min** | **10 min** | **15 min** | **0.5 h** | **3 h** | **4 h** |
| g1 | - | -10.84 | -31.37 | -40.51 | -39.63 | -13.31 | -1.86 |
| g2 | - | -10.77 | -39.03 | -43.79 | -40.41 | -13.33 | -1.79 |
| g3 | - | -10.23 | -37.38 | -39.61 | -36.27 | -15.43 | 1.91 |
| g4 | - | -13.56 | -38.63 | -39.89 | -39.05 | -19.19 | 1.36 |
| g5 | - | -10.02 | -39.71 | -45.75 | -40.47 | 1.47 | 3.10 |
| g6 | - | -9.84 | -39.67 | -41.39 | -40.87 | -6.80 | 1.99 |
| Average value | - | -10.87 | -37.63 | -41.82 | -39.45 | -11.10 | 0.78 |
| SD | - | 1.37 | 3.18 | 2.45 | 1.69 | 7.36 | 2.10 |

**Table 1-6: Percentage changes in pupil diameter before and after administration of 1.75% JQ-6 solution in six rabbits**

| **No.** | **Percentage change (%)** | | | | | | |
|---|---|---|---|---|---|---|---|
| | **-0.5 h** | **5 min** | **10 min** | **15 min** | **0.5 h** | **3 h** | **4 h** |
| d1 | - | -16.45 | -37.16 | -43.93 | -41.17 | -11.08 | 0.40 |
| d2 | - | -9.21 | -44.07 | -50.30 | -46.50 | -17.65 | -0.50 |
| d3 | - | -9.93 | -32.07 | -36.57 | -35.05 | -6.95 | -0.92 |
| d4 | - | -18.36 | -44.23 | -49.50 | -47.81 | -12.34 | -0.45 |
| d5 | - | -12.49 | -37.19 | -43.41 | -42.82 | -6.91 | -0.05 |
| d6 | - | -14.04 | -44.10 | -45.41 | -44.78 | -22.91 | 0.52 |
| Average value | - | -13.41 | -39.80 | -44.85 | -43.02 | -12.97 | -0.17 |
| SD | - | 3.60 | 5.10 | 4.96 | 4.58 | 6.29 | 0.56 |

**Table 1-7: Percentage changes in pupil diameter before and after administration of 1.81% JQ-8 solution in six rabbits**

| **No.** | **Percentage change (%)** | | | | | | |
|---|---|---|---|---|---|---|---|
| | **-0.5 h** | **5 min** | **10 min** | **15 min** | **0.5 h** | **3 h** | **4 h** |
| e1 | - | -10.02 | -35.68 | -40.77 | -43.69 | -11.98 | -1.75 |
| e1 | - | -13.95 | -39.76 | -43.15 | -40.97 | -14.66 | -0.71 |
| e3 | - | -11.90 | -33.63 | -42.68 | -39.89 | -13.81 | -0.83 |
| e4 | - | -12.87 | -39.82 | -40.87 | -34.82 | -10.12 | -0.39 |
| e5 | - | -9.70 | -39.02 | -45.05 | -38.82 | 0.72 | -1.33 |
| e6 | - | -7.76 | -38.00 | -45.97 | -41.23 | -9.01 | 3.70 |
| Average value | - | -11.03 | -37.65 | -43.08 | -39.90 | -9.81 | -0.22 |
| SD | - | 2.29 | 2.50 | 2.12 | 2.98 | 5.58 | 1.98 |

**Table 1-8: Percentage changes in pupil diameter before and after administration of 1.77% JQ-9 solution in six rabbits**

| **No.** | **Percentage change (%)** | | | | | | |
|---|---|---|---|---|---|---|---|
| | **-0.5 h** | **5 min** | **10 min** | **15 min** | **0.5 h** | **3 h** | **4 h** |
| f1 | - | -14.89 | -34.12 | -39.45 | -40.70 | -11.88 | -0.84 |
| f2 | - | -9.98 | -42.35 | -43.39 | -40.65 | -15.58 | 0.49 |
| f3 | - | -20.31 | -36.41 | -38.81 | -33.84 | -10.81 | -0.44 |
| f4 | - | -20.68 | -44.05 | -45.69 | -40.31 | -13.10 | 0.79 |
| f5 | - | -21.86 | -44.02 | -44.38 | -45.00 | -16.19 | -0.36 |
| f6 | - | -27.67 | -39.21 | -45.14 | -43.66 | -12.95 | 0.66 |
| Average value | - | -19.23 | -40.03 | -42.81 | -40.69 | -13.42 | 0.05 |
| SD | - | 6.09 | 4.15 | 2.96 | 3.86 | 2.09 | 0.68 |

**Table 1-9: Percentage changes in pupil diameter before and after administration of 1.82% JQ-10 solution in six rabbits**

| **No.** | **Percentage change (%)** | | | | | | |
|---|---|---|---|---|---|---|---|
| | **-0.5 h** | **5 min** | **10 min** | **15 min** | **0.5 h** | **3 h** | **4 h** |
| h1 | - | -14.09 | -34.16 | -39.42 | -36.64 | -7.79 | 2.27 |
| h2 | - | -6.50 | -29.03 | -44.58 | -42.83 | -21.95 | -8.88 |
| h3 | - | -11.55 | -45.58 | -49.02 | -45.05 | -27.25 | -10.48 |
| h4 | - | -11.53 | -41.35 | -47.37 | -44.04 | -21.72 | -6.21 |
| h5 | - | -12.72 | -50.54 | -43.71 | -38.53 | -21.04 | -11.03 |
| h6 | - | -27.12 | -48.11 | -44.89 | -41.57 | -14.30 | -7.81 |
| Average value | - | -13.92 | -41.46 | -44.83 | -41.44 | -19.01 | -7.03 |
| SD | - | 6.96 | 8.38 | 3.30 | 3.26 | 6.87 | 4.88 |

**Table 1-10: Percentage changes in pupil diameter (%) before and after administration in each group (x̅ ± SD value)**

| **Compound** | **Number of animals** | **Percentage change in pupil diameter (%)** | | | | | |
|---|---|---|---|---|---|---|---|
| | | **5 min** | **10 min** | **15 min** | **0.5 h** | **3 h** | **4 h** |
| 1.25% pilocarpine hydrochloride | 6 | -7.81±4.48 | -29.35±5.08 | -35.33±6.18 | -34.59±5.84 | -3.13±7.35 | 0.25±2.14 |
| 1.82% JQ-1 | 6 | -17.53±6.67 | -42.23±7.72 | -44.46±5.59 | -40.50±4.65 | -17.25±10.99 | -6.47±9.66 |
| 1.90% JQ-2 | 6 | -12.87±5.05 | -40.50±6.00 | -43.07±5.25 | -40.07±5.56 | -12.40±5.71 | 0.17±1.12 |
| 1.75%JQ-3 | 6 | -16.45±10.53 | -43.22±7.24 | -46.15±3.98 | -43.85±4.42 | -12.67±6.47 | 1.92±4.48 |
| 0.91% JQ-5 | 6 | -10.87±1.37 | -37.63±3.18 | -41.82±2.45 | -39.45±1.69 | -11.10±7.36 | 0.78±2.10 |
| 1.75%JQ-6 | 6 | -13.41±3.60 | -39.80±5.10 | -44.85±4.96 | -43.02±4.58 | -12.97±6.29 | -0.17±0.56 |
| 1.81%JQ-8 | 6 | -11.03±2.29 | -37.65±2.50 | -43.08±2.12 | -39.90±2.98 | -9.81±5.58 | -0.22±1.98 |
| 1.77%JQ-9 | 6 | -19.23±6.09 | -40.03±4.15 | -42.81±2.96 | -40.69±3.86 | -13.42±2.09 | 0.05±0.68 |
| 1.82%JQ-10 | 6 | -13.92±6.96 | -41.46±8.38 | -44.83±3.30 | -41.44±3.26 | -19.01±6.87 | -7.03±4.88 |

From Table 1-10, it can be seen that the pilocarpine benzoic acid series compounds of the present disclosure overall exhibit a superior pupil constriction effect compared to the pilocarpine hydrochloride. The details are as follows: 1) the pilocarpine benzoic acid series compounds of the present disclosure exhibited a superior initial pupil constriction effect at 5 min compared to the pilocarpine hydrochloride, achieving a faster onset of effect; 2) the measured value of the pilocarpine hydrochloride solution at the time point of 3 h was -3.13 ± 7.35, indicating that the solution had substantially no pupil constriction effect; however, the series compounds of the present application still exhibited a significant pupil constriction effect when measured at the time point of 3 h, with the effect lasting for 4 h or even longer, and the statistical results show that the duration of effect was extended by no less than 33% compared to the pilocarpine hydrochloride; 3) the maximum percentage change in pupil diameter caused by using pilocarpine hydrochloride was only around 35%, whereas the maximum percentage change in pupil diameter caused by using the series compounds of the present application could reach 40%-45%, with the maximum strength of effect enhanced by 15%-30%. It can be seen that the pilocarpine benzoic acid series compounds of the present disclosure exhibited a faster onset of effect, longer duration of effect, and greater strength of effect, with statistically significant differences.

As can be seen from the comparison results of the irritation to rabbit eyes caused by pilocarpine hydrochloride solution (FIG. 1) and the pilocarpine substituted benzoic acid series solutions (FIGs. 2-9), the irritation caused by the pilocarpine substituted benzoic acid series compounds of the present disclosure was equivalent to that caused by the hydrochloride.

### 2.2. Test results

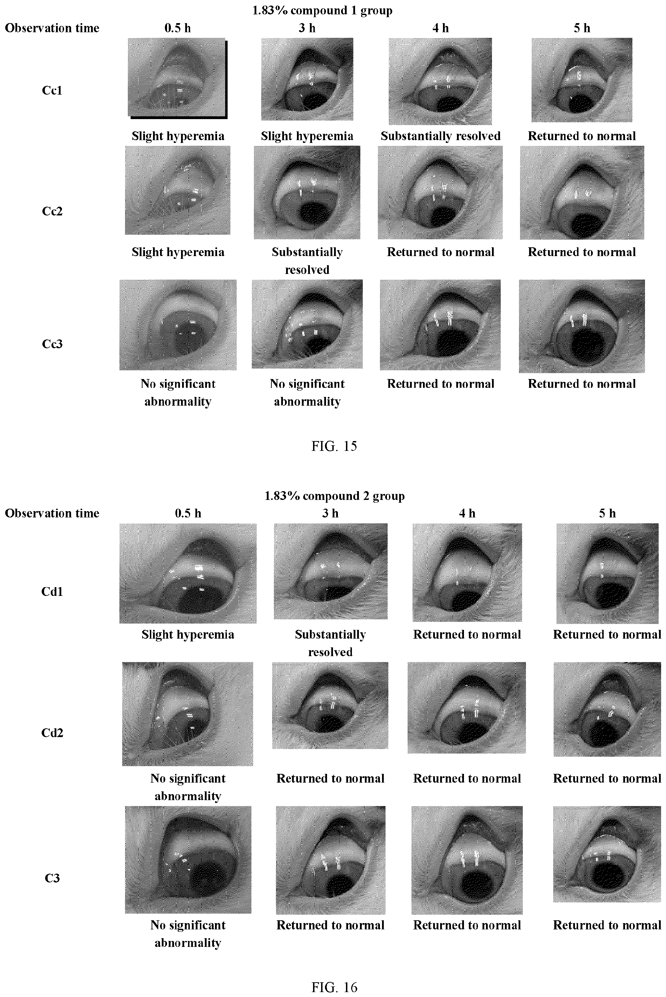

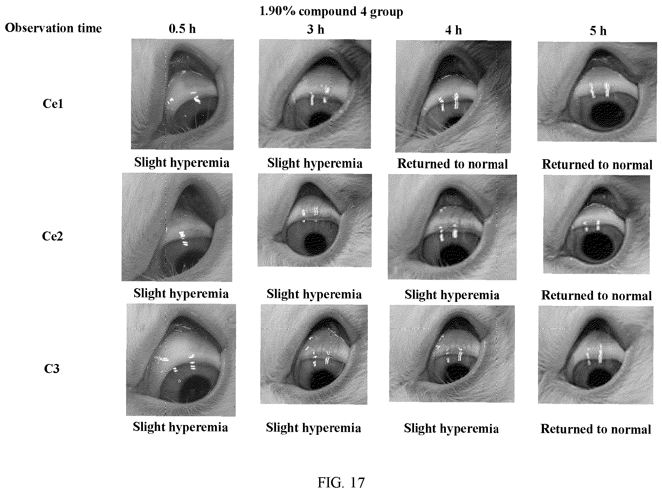

The results of the irritation to rabbit eyes caused by different eye drop solutions of the phenylacetic acid series compounds over different time periods are shown in FIGs. 15-17, and the test results of rabbit pupil change values with different eye drop solutions are shown in Tables 1-11, 1-12 and 1-13.

**Table 1-11: Percentage changes in pupil diameter before and after administration of 1.83% compound 1 solution in five rabbits**

| **No.** | **Percentage change in pupil diameter caused by 1.83% compound 1 solution** | |
|---|---|---|
| | **3 h** | **4 h** |
| Cc1 | -17.48% | -3.29% |
| Cc2 | -14.47% | -0.69% |
| Cc3 | -18.84% | -2.24% |
| Cc4 | -11.57% | -3.73% |
| Cc5 | -23.80% | -7.32% |
| Average value | -17.23% | -3.45% |
| SD | 0.05 | 0.02 |

**Table 1-12: Percentage changes in pupil diameter before and after administration of 1.83% compound 2 solution in five rabbits**

| **No.** | **Percentage change in pupil diameter caused by 1.83% compound 2 solution** | |
|---|---|---|
| | **3 h** | **4 h** |
| Cd1 | -8.78% | -0.56% |
| Cd2 | -12.39% | -3.06% |
| Cd3 | -11.40% | -0.22% |
| Cd4 | -10.20% | -1.70% |
| Cd5 | -17.06% | -6.24% |
| Average value | -11.97% | -2.35% |
| SD | 0.03 | 0.02 |

**Table 1-13: Percentage changes in pupil diameter before and after administration of 1.90% compound 4 solution in five rabbits**

| **No.** | **Percentage change in pupil diameter caused by 1.90% compound 4 solution** | |
|---|---|---|
| | **3 h** | **4 h** |
| Ce1 | -12.16% | 0.22% |
| Ce2 | -5.79% | 0.83% |
| Ce3 | -10.24% | -0.80% |
| Ce4 | -13.14% | 0.19% |
| Ce5 | -9.10% | -0.52% |
| Average value | -10.08% | -0.02% |
| SD | 0.03 | 0.01 |

### Test Example 2

Reagent: normal saline (specification: 500 mL:4.5 g), purchased from Jiangxi Kelun Pharmaceutical Co., Ltd.

### Solution preparation:

Pilocarpine hydrochloride: with a molecular weight of 244.72 and a purity greater than 95%, from Nanjing Jiqun Pharmaceutical Technology Co., Ltd., prepared into a 1.25% solution using normal saline.

Pilocarpine nitrate: with a molecular weight of 271.27 and a purity of 98%, from Nanjing Jiqun Pharmaceutical Technology Co., Ltd., prepared into a 1.38% solution using normal saline.

Pilocarpine salicylate: with a molecular weight of 346.24 and a purity of 99.73%, from Nanjing Jiqun Pharmaceutical Technology Co., Ltd., prepared into a 1.76% solution using normal saline.

Pilocarpine malate: with a molecular weight of 342.21 and a purity of 99.72%, from Nanjing Jiqun Pharmaceutical Technology Co., Ltd., prepared into a 1.74% solution using normal saline.

Pilocarpine maleate: with a molecular weight of 324.19 and a purity of 99.76%, from Nanjing Jiqun Pharmaceutical Technology Co., Ltd., prepared into a 1.65% solution using normal saline.

Pilocarpine fumarate: with a molecular weight of 324.19 and a purity of 99.74%, from Nanjing Jiqun Pharmaceutical Technology Co., Ltd., prepared into a 1.65% solution using normal saline.

Pilocarpine lipoate: with a molecular weight of 414.58 and a purity of 99.56%, from Nanjing Jiqun Pharmaceutical Technology Co., Ltd., prepared into a 2.11% solution using normal saline.

Note: The concentrations of the compounds were different, but they all contained an equimolar amount of pilocarpine.

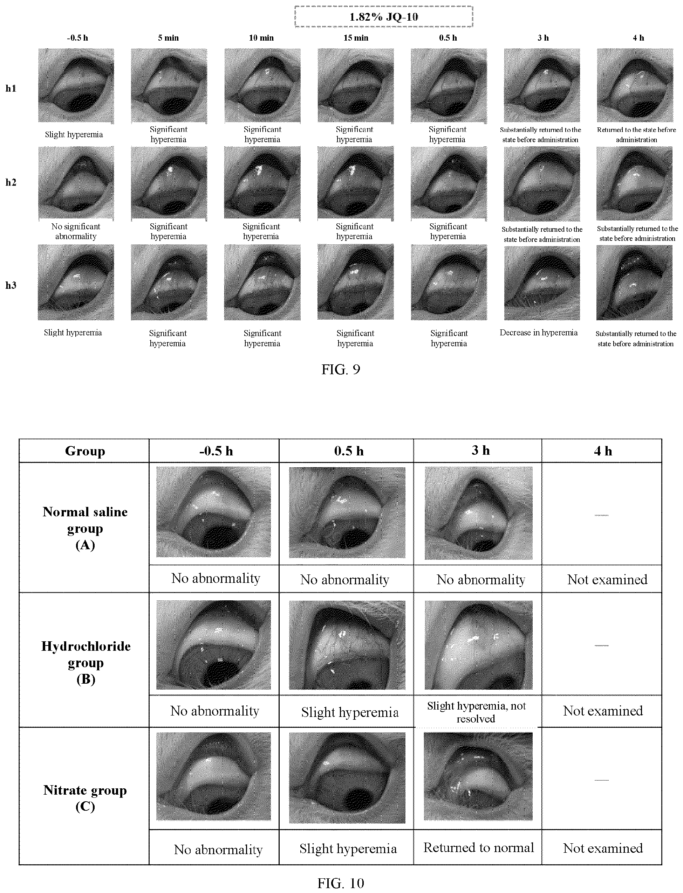

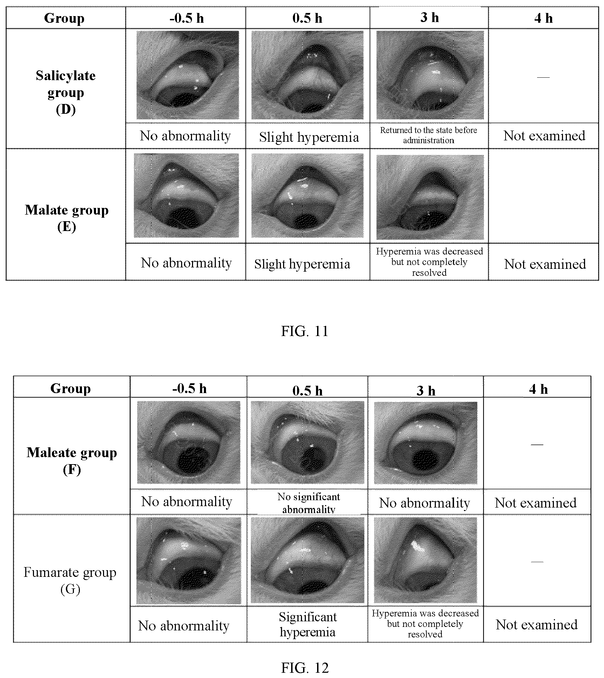

Each rabbit in each group was subjected to single eye drop administration in the left eye, with an administration volume of 100 µL. The pupil and iris diameters of the left eye of the rabbits were measured at time points of -0.5 h (i.e., half an hour before administration), 0.5 h, 3 h, and 4 h relative to administration (observation time points were extended if necessary), with the measurement performed 5 times for each rabbit at each time point. The entire measurement process was completed by the same person. The measurement data are shown in Tables 2-1 and 2-2. At each measurement time point, the eye irritation condition in each group was examined concurrently with the pupil and iris diameters recorded by photography, and the observed phenomena were noted in the original records, as detailed in FIGs. 10 to 12. (Note: The administration of lipoate caused significant irritation and noticeable hyperemia, and the hyperemia exhibited prolonged duration and was slow to resolve. Additionally, the rabbits emitted stress-induced screams when the lipoate was administered).

**Table 2-1: Percentage changes in pupil diameter (%) before and after administration in each group (average value ± SD)**

| **Group** | **Drug concentration (%)** | **Number of animals (n)** | **Percentage change in pupil diameter (%)** | | | |
|---|---|---|---|---|---|---|
| | | | **Before administration** | **0.5 h** | **3 h** | **4 h** |
| Normal saline | - | 3 | / | 3.37±2.10 | 1.69±1.79 | - |
| Pilocarpine hydrochloride | 1.25 | 3 | / | -32.46±1.69 | 2.07±3.90 | - |
| Pilocarpine nitrate | 1.38 | 3 | / | -32.03±2.72 | -0.27±1.25 | - |
| Pilocarpine salicylate | 1.76 | 3 | / | -30.12±4.47 | 2.91±1.07 | - |
| Pilocarpine malate | 1.74 | 3 | / | -27.71±4.98 | 0.07±2.49 | - |
| Pilocarpine maleate | 1.65 | 3 | / | -27.53±4.42 | -1.52±6.38 | - |
| Pilocarpine fumarate | 1.65 | 3 | / | -22.09±6.44 | -1.00±1.51 | - |
| Pilocarpine lipoate | 2.11 | 5 | / | -40.69±2.68 | -3.11±2.38 | - |

In the above table, "/" indicates that the change in pupil contraction was not determined before administration; "-" indicates that it was not detected.

It can be seen by a comprehensive comparison of the data in Table 2-1 above and Table 1-10 in Test Example 1 that, the overall effect of the pilocarpine benzoic acid series compounds of the present disclosure was stronger than that of the pilocarpine hydrochloride, the pilocarpine nitrate, the pilocarpine salicylate, and the like. Correspondingly, the pilocarpine benzoic acid series compounds exhibited a longer duration of effect and relatively more sustained efficacy.

As can be seen from the determination results in Table 2-1 above and Tables 1-11, 1-12, and 1-13 in Test Example 1, the pilocarpine hydrochloride, the pilocarpine nitrate, the pilocarpine salicylate, the pilocarpine malate, the pilocarpine maleate, the pilocarpine fumarate, and the like showed substantially no miosis effect on rabbit pupils 3 h after administration, whereas the compound 1 solution in Table 1-11, the compound 2 solution in Table 1-12, and the compound 4 solution in Table 1-13 still showed miosis effect 3 h after administration. It can be seen that compared to the pilocarpine salt series in Table 2-1, the duration of effect of the pilocarpine phenylacetate series was extended by at least 1 h, and the duration of efficacy was relatively extended by no less than 33%, thereby increasing the duration of efficacy and reducing the frequency of administration.

### Test Example 3

Under the condition of normal saline, the onset time, strength of effect, duration of effect, and irritation of 1.25% pilocarpine hydrochloride and different concentrations (0.88%, 1.75%, 3.50%) of pilocarpine p-toluate were tested.

### (I) Test grouping

| **No.** | **Compound** | **Concentration (%)** | **Purity** | **Number of animals** |
|---|---|---|---|---|
| 1 | Pilocarpine hydrochloride | 1.25 | 95% | 6 |
| 2 | Pilocarpine *p*-toluate | 0.88 | 95% | 6 |
| 3 | Pilocarpine *p*-toluate | 1.75 | 95% | 6 |
| 4 | Pilocarpine *p*-toluate | 3.50 | 95% | 6 |

| | | | | |
|---|---|---|---|---|
| Note: The 1.75% pilocarpine p-toluate contained the same molar concentration of pilocarpine as the 1.25% pilocarpine hydrochloride, and the solutions of the above groups were all prepared using normal saline (specification: 500 mL:4.5 g, purchased from Jiangxi Kelun Pharmaceutical Co., Ltd.). | | | | |

### (II) Experiment

### (1) Route of administration

Each rabbit was subjected to eye drop administration in the left eye (using a pipette), with an administration volume of 100 µL.

### (2) Test arrangement

Number of animals: 6 rabbits per compound.
Administration schedule: single administration.

Measurement indicators and time points:
the pupil diameter of the left eye of the rabbits was measured at time points of -0.5 h (i.e., half an hour before administration), 5 min, 10 min, 15 min, 0.5 h, 3 h and 4 h relative to administration, with the measurement performed 3 times for each rabbit at each time point.
Irritation: examined concurrently with the pupil diameter.

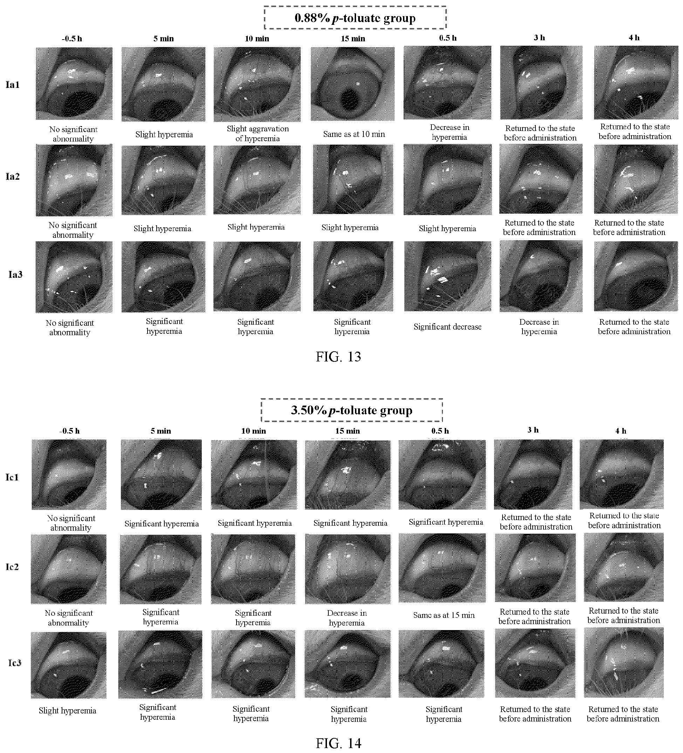

The numerical results of pupil changes are shown in Tables 3-1 to 3-4, and the changes in eye irritation phenomenon are shown in FIGs. 13 and 14.

**Table 3-1: Percentage changes in pupil diameter (%) before and after administration of 1.25% pilocarpine hydrochloride in 6 rabbits**

| **No.** | **Percentage change (%)** | | | | | | |
|---|---|---|---|---|---|---|---|
| | **-0.5 h** | **5 min** | **10 min** | **15 min** | **0.5 h** | **3 h** | **4 h** |
| L1 | - | -6.51 | -26.64 | -33.71 | -32.89 | -6.71 | 2.49 |
| L2 | - | -15.75 | -36.43 | -42.10 | -39.82 | -16.22 | -2.39 |
| L3 | - | -2.17 | -21.69 | -24.95 | -23.64 | 0.16 | 0.54 |
| L4 | - | -6.28 | -29.40 | -40.68 | -36.45 | 4.54 | -0.15 |
| L5 | - | -7.23 | -32.93 | -36.99 | -36.62 | -0.58 | -1.79 |
| L6 | - | -8.93 | -28.98 | -33.54 | -38.14 | 0.03 | 2.79 |
| Average value | - | -7.81 | -29.35 | -35.33 | -34.59 | -3.13 | 0.25 |
| SD | - | 4.48 | 5.08 | 6.18 | 5.84 | 7.35 | 2.14 |

**Table 3-2: Percentage changes in pupil diameter (%) before and after administration of 0.88% pilocarpine p-toluate in 6 rabbits**

| **No.** | **Percentage change (%)** | | | | | | |
|---|---|---|---|---|---|---|---|
| | **-0.5 h** | **5 min** | **10 min** | **15 min** | **0.5 h** | **3 h** | **4 h** |
| Ia1 | - | -9.41 | -48.90 | -43.68 | -37.28 | -7.91 | -0.54 |
| Ia2 | - | -12.03 | -41.07 | -50.07 | -44.65 | -14.57 | -1.39 |
| Ia3 | - | -13.93 | -28.39 | -30.13 | -28.71 | -2.85 | 0.26 |
| Ia4 | - | -4.80 | -33.75 | -43.40 | -36.60 | -9.65 | 3.67 |
| Ia5 | - | -6.70 | -34.62 | -41.17 | -41.12 | -14.66 | -2.65 |
| Ia6 | - | -13.54 | -30.54 | -36.73 | -36.25 | -12.43 | 4.83 |
| Average value | - | -10.07 | -36.21 | -40.86 | -37.44 | -10.34 | 0.70 |
| SD | - | 3.75 | 7.57 | 6.81 | 5.37 | 4.55 | 2.94 |

**Table 3-3: Percentage changes in pupil diameter (%) before and after administration of 1.75% pilocarpine p-toluate in 6 rabbits**

| **No.** | **Percentage change (%)** | | | | | | |
|---|---|---|---|---|---|---|---|
| | **-0.5 h** | **5 min** | **10 min** | **15 min** | **0.5 h** | **3 h** | **4 h** |
| d1 | - | -16.45 | -37.16 | -43.93 | -41.17 | -11.08 | 0.40 |
| d2 | - | -9.21 | -44.07 | -50.30 | -46.50 | -17.65 | -0.50 |
| d3 | - | -9.93 | -32.07 | -36.57 | -35.05 | -6.95 | -0.92 |
| d4 | - | -18.36 | -44.23 | -49.50 | -47.81 | -12.34 | -0.45 |
| d5 | - | -12.49 | -37.19 | -43.41 | -42.82 | -6.91 | -0.05 |
| d6 | - | -14.04 | -44.10 | -45.41 | -44.78 | -22.91 | 0.52 |
| Average value | - | -13.41 | -39.80 | -44.85 | -43.02 | -12.97 | -0.17 |
| SD | - | 3.60 | 5.10 | 4.96 | 4.58 | 6.29 | 0.56 |

**Table 3-4: Percentage changes in pupil diameter (%) before and after administration of 3.50% pilocarpine p-toluate**

| **No.** | **Percentage change (%)** | | | | | | |
|---|---|---|---|---|---|---|---|
| | **-0.5 h** | **5 min** | **10 min** | **15 min** | **0.5 h** | **3 h** | **4 h** |
| Ic1 | - | -17.29 | -36.06 | -41.19 | -39.24 | -11.88 | 0.57 |
| Ic2 | - | -15.26 | -45.62 | -47.24 | -43.99 | -18.24 | 0.22 |
| Ic3 | - | -25.35 | -33.89 | -36.41 | -32.38 | -0.16 | -0.59 |
| Ic4 | - | -19.35 | -50.03 | -49.92 | -45.25 | -21.18 | 1.32 |
| Ic5 | - | -26.00 | -49.43 | -49.70 | -45.25 | -20.59 | 0.87 |
| Ic6 | - | -30.85 | -49.04 | -50.66 | -46.35 | -29.99 | -5.52 |
| Average value | - | -22.35 | -44.01 | -45.85 | -42.08 | -17.00 | -0.52 |
| SD | - | 5.99 | 7.20 | 5.78 | 5.37 | 10.10 | 2.53 |

As can be seen from the data in the above table, compared to the 1.25% pilocarpine hydrochloride group, the 0.88% pilocarpine p-toluate (in which the molar concentration of pilocarpine was only half of that in the 1.25% pilocarpine hydrochloride) demonstrated increased strength of effect at each time point. It can be seen that a better effect was already achieved when the drug concentration was halved, so that the administration dose could be significantly reduced, which correspondingly reduced the drug's side effects and the associated impurities. For the 1.75% pilocarpinep-toluate (in which the molar concentration of pilocarpine was the same as that in the 1.25% pilocarpine hydrochloride), the increase in strength of effect at each time point was more significant compared to the 1.25% pilocarpine hydrochloride. For the 3.50% pilocarpine p-toluate (in which the molar concentration of pilocarpine was 2 times that in the 1.25% pilocarpine hydrochloride), the strength of effect at each time point was also significantly enhanced compared to the 1.25% pilocarpine hydrochloride.

FIGs. 13 and 14 respectively show the images of irritation to rabbit eyes caused by pilocarpine p-toluate at concentrations of 0.88% and 3.50%. As can be seen from the images, the irritation to rabbit eyes caused by the 0.88% pilocarpine p-toluate was significantly lower than that caused by the 3.50% pilocarpinep-toluate.

### Test Example 4

Reagents: normal saline (specification: 500 mL:4.5 g), purchased from Jiangxi Kelun Pharmaceutical Co., Ltd.;

compound 1 and compound 2 (from Nanjing Jiqun Pharmaceutical Technology Co., Ltd.);
1) a compound 1 solution at a concentration of 0.915% prepared using normal saline;
2) a compound 2 solution at a concentration of 0.915% prepared using normal saline.

The miosis effects of the 0.915% compound 1 solution and the 0.915% compound 2 solution were determined using the method in Test Example 2, and the determination results are shown in Tables 4-1 and 4-2 below.

**Table 4-1: Percentage changes in pupil diameter before and after administration of 0.915% compound 1 solution in five rabbits**

| **No.** | **Percentage change in pupil diameter caused by 0.915% compound 1 solution** | |
|---|---|---|
| | **Before administration** | **3 h** |
| Cb1 | - | 0.51% |
| Cb2 | - | -0.98% |
| Cb3 | - | -0.17% |
| Cb4 | - | -0.37% |
| Cb5 | - | -1.00% |
| Average value | - | -0.40% |
| SD | - | 0.01 |

**Table 4-2: Percentage changes in pupil diameter before and after administration of 0.915% compound 2 solution in five rabbits**

| **No.** | **Percentage change in pupil diameter caused by 0.915% compound 2 solution** | |
|---|---|---|
| | **Before administration** | **3 h** |
| Az1 | - | -2.24% |
| Az2 | - | -0.32% |
| Az3 | - | 1.47% |
| Az4 | - | -0.23% |
| Az5 | - | -7.89% |
| Average value | - | -1.84% |
| SD | - | 0.04 |

From the comparison of the data results in Test Example 2 and this test example, it can be seen that the miosis effects of compound 1 and compound 2 were comparable to the effect of pilocarpine hydrochloride at half of the concentration of the pilocarpine hydrochloride, indicating that the pilocarpine phenylacetic acid derivative salts can reduce the administration concentration.

### Test Example 5

### Formulation Components and Efficacy Test

Formulation 1: a specific amount of compound 1, boric acid, sodium citrate and sodium chloride was taken, and an appropriate amount of sodium hydroxide/hydrochloric acid was added (for pH adjustment), thus preparing a formulation solution with a concentration of 18.3 mg/mL (10.6 mg/mL in terms of pilocarpine) for compound 1, 10 mg/mL for boric acid, 0.15 mg/mL for sodium citrate and 1 mg/mL for sodium chloride and a pH value of 4.5.

Formulation 2: a specific amount of compound 1, boric acid, sodium citrate and sodium chloride was taken, and an appropriate amount of sodium hydroxide/hydrochloric acid was added (for pH adjustment), thus preparing a formulation solution with a concentration of 9.15 mg/mL (5.3 mg/mL in terms of pilocarpine) for compound 1, 10 mg/mL for boric acid, 0.15 mg/mL for sodium citrate and 1 mg/mL for sodium chloride and a pH value of 4.5.

Formulation 3: a specific amount of compound 2, boric acid, sodium citrate and sodium chloride was taken, and an appropriate amount of sodium hydroxide/hydrochloric acid was added (for pH adjustment), thus preparing a formulation solution with a concentration of 18.3 mg/mL (10.6 mg/mL in terms of pilocarpine) for compound 2, 10 mg/mL for boric acid, 0.15 mg/mL for sodium citrate and 1 mg/mL for sodium chloride and a pH value of 4.5.

Formulation 4: a specific amount of compound 2, boric acid, sodium citrate and sodium chloride was taken, and an appropriate amount of sodium hydroxide/hydrochloric acid was added (for pH adjustment), thus preparing a formulation solution with a concentration of 9.15 mg/mL (5.3 mg/mL in terms of pilocarpine) for compound 2, 10 mg/mL for boric acid, 0.15 mg/mL for sodium citrate and 1 mg/mL for sodium chloride and a pH value of 4.5.

Formulation 5: a specific amount of pilocarpine hydrochloride compound, boric acid, sodium citrate and sodium chloride was taken, and an appropriate amount of sodium hydroxide/hydrochloric acid was added (for pH adjustment), thus preparing a formulation solution with a concentration of 12.5 mg/mL (10.6 mg/mL in terms of pilocarpine) for pilocarpine hydrochloride, 10 mg/mL for boric acid, 0.15 mg/mL for sodium citrate and 1 mg/mL for sodium chloride and a pH value of 4.5. Formulations 1, 3 and 5 contained the same molar concentration of pilocarpine, and formulations 2 and 4 contained pilocarpine at half of the molar concentration of formulations 1, 3 and 5.

The miosis effects of the solutions of formulations 1-5 were determined using the determination method in Test Example 2, and the determination results are shown in Table 5-1 below.

**Table 5-1: Percentage changes in pupil diameter before and after administration of solutions of formulations 1-5 in five rabbits (average value ± SD)**

| **Group** | **Number of animals** | **Percentage change in pupil diameter (%)** | | |
|---|---|---|---|---|
| | | **3h** | **4 h** | **Note** |
| Formulation 1 | 5 | -10±0.04 | -0.8±0.03 | The duration of the miosis effect reached 3 h in all rabbits, 4 h in 4 rabbits and 5 h in 1 rabbit. |
| Formulation 3 | 5 | -11±0.02 | -0.13±0.01 | The duration of the miosis effect reached 4 h in all rabbits. |
| Formulation 2 | 5 | -2±0.01 | 2±0.02 | The duration of the miosis effect reached 4 h in all rabbits. |
| Formulation 4 | 5 | -2±0.04 | 0±0.03 | The duration of the miosis effect reached 3 h in all rabbits and 4 h in 1 rabbit. |
| Formulation 5 | 5 | -1±0.05 | 0.25±0.01 | The duration of the miosis effect reached 3 h in 4 rabbits, while 1 rabbit showed no miosis effect at 3 h. |

As can be seen by comparison of the data in Table 5-1, according to the effects of formulations 1, 3 and 5 at the same concentration of pilocarpine, the duration of effect of formulations 1 and 3 was extended by no less than 1 h compared to formulation 5, and the effects of formulations 2 and 4 at half of the molar concentration of formulation 5 were comparable to that of formulation 5, with some aspects being superior to the effect of formulation 5. In conclusion, the duration of the miosis effect of the pilocarpine phenylacetic acid series formulations of the present disclosure was significantly superior to that of the pilocarpine hydrochloride product on the market.

### Test Example 6

### Stability Tests

### (1) Stability tests on compound starting materials

Samples: compound 1, compound 2 and pilocarpine hydrochloride starting material solid particles.

High-temperature test: 1 g of each of compound 1, compound 2 and pilocarpine hydrochloride starting materials were taken and placed in an open container inside a constant temperature device at 40 °C; samples were taken at time points of day 0 and day 5, and then tested according to the key stability examination items. The stability of the active pharmaceutical ingredient itself and the trend of changes in stability under the conditions of influencing factor tests were observed.

Strong light irradiation test: 1 g of each of compound 1, compound 2 and pilocarpine hydrochloride starting materials were taken and placed in an open container inside a light irradiation box, with the illumination set at 4500 lx ± 500 lx; samples were taken at time points of day 0 and day 5, and then tested according to the key stability examination items.

High-humidity test: 1 g of each of compound 1, compound 2 and pilocarpine hydrochloride starting materials was taken and placed in an open container inside a constant humidity closed container at 25 °C and with a relative humidity of 75% ± 5%; samples were taken at day 0 and day 5, and then tested according to the requirements of the key stability examination items.

According to the records in the European Pharmacopoeia (EP), the key related substances to be examined for pilocarpine include: impurity A isopilocarpine (Z-A), impurity B pilocarpic acid (Z-B), and impurity C isopilocarpic acid (Z-C). The samples taken at different time points from the above high-temperature test, strong light irradiation test and high-humidity test were separately tested using high-performance liquid chromatography to observe the trend of changes in stability under the conditions of the influencing factor tests.

The related substances were determined according to the high-performance liquid chromatography (General Chapter 0512, Chinese Pharmacopoeia, Volume IV, 2020 Edition).

Test sample solution: an appropriate amount of each test sample was precisely weighed, dissolved in water, and diluted to prepare a solution containing 1 mg of test sample per 1 mL.

Chromatographic conditions: octadecyl bonded silica gel was used as a filler [kromasil 100-5-C18 column (4.0 mm × 125 m) or a chromatographic column with equivalent performance], 0.679 g/L tetrabutylammonium dihydrogen phosphate-methanol-acetonitrile (885:55:60) (adjusted to pH 7.7 with dilute aqueous ammonia) was used as mobile phase A, and acetonitrile-water (80:20) was used as mobile phase B; gradient elution was performed according to Table 6-1, with a column temperature of 30 °C, a flow rate of 1.0 mL/min, a detection wavelength of 220 nm, and a sample injection volume of 20 µL.

**Table 6-1**

| **Time (min)** | **Mobile phase A (%)** | **Mobile phase B (%)** |
|---|---|---|
| 0 | 100 | 0 |
| 35 | 100 | 0 |
| 40 | 20 | 80 |
| 43 | 20 | 80 |
| 45 | 100 | 0 |
| 70 | 100 | 0 |

The tests were performed according to the above methods, and the test results are shown in Table 6-2.

**Table 6-2: Results of stability tests on starting materials**

| **Compound** | **Influencing factor** | **(Z-B) (%)** | **(Z-C) (%)** | **(Z-A) (%)** | **Maximum single impurity (%)** | **Total impurity (%)** |
|---|---|---|---|---|---|---|
| Compound 1 | Day 0 | 0 | 0 | 0.328 | 0 | 0.328 |
| | 40 °C-5 days | 0 | 0 | 0.365 | 0 | 0.365 |
| | Light-5 days | 0 | 0 | 0.329 | 0 | 0.329 |
| | RH 75%-5 days | 0 | 0 | 0.310 | 0 | 0.331 |
| Compound 2 | Day 0 | 0 | 0 | 0.307 | 0 | 0.307 |
| | 40 °C-5 days | 0.072 | 0 | 0.338 | 0 | 0.353 |
| | Light-5 days | 0.046 | 0 | 0.333 | 0 | 0.339 |
| | RH 75%-5 days | 0.116 | 0 | 0.311 | 0 | 0.427 |
| Pilocarpine hydrochloride | Day 0 | 0 | 0 | 0.197 | 0 | 0.197 |
| | 40 °C-5 days | 0 | 0 | 0.248 | 0 | 0.248 |
| | Light-5 days | 0 | 0 | 0.248 | 0 | 0.248 |
| | RH 75%-5 days | 1.641 | 0 | 0.259 | 0.123 | 2.023 |

As can be seen by comparison of the data in the above table, under the conditions of 40 °C and high light irradiation, the impurity increasing rates in the starting materials of compound 1 and compound 2 were comparable to those of the pilocarpine hydrochloride starting material; however, when being placed 5 days under the condition of high humidity (RH 75%), the impurity increasing rates of the starting materials of compound 1 and compound 2 were significantly slower than those of the pilocarpine hydrochloride. Overall, the stability of the pilocarpine phenylacetic acid series starting materials of the present disclosure was superior to that of pilocarpine hydrochloride.

### (2) Stability tests on formulations

Stability tests were performed on the above formulations 1, 3 and 5:
(1) Formulations 1, 3 and 5 were separately taken and placed in an open container under the condition of a constant temperature of 40 °C; samples were taken at time points of day 0 and day 5, and the impurity increment in solutions at day 10 was tested according to the key stability examination items. The stability of the formulation itself and the trend of changes in stability under the conditions of influencing factor tests were observed.
(2) Formulations 1, 3 and 5 were separately taken and placed in an open container under the conditions of a constant temperature of 40 °C and a constant humidity of RH 25%, and the impurity increment in solutions at day 5 and day 10 was tested according to the key stability examination items. The stability of the formulation itself and the trend of changes in stability under the conditions of influencing factor tests were observed. The test results are shown in Table 6-3 below.

**Table 6-3: Results of stability tests on different formulations**

| **Examination condition** | **Formulation** | **Time** | **Impurity B increment (%)** | **Impurity C increment (%)** | **Impurity A increment (%)** | **Maximum single impurity increment (%)** | **Total impurity increment (%)** | **Total impurity content (%)** |
|---|---|---|---|---|---|---|---|---|
| 40 °C | Formulation 5 | 10 d | 1.03 | 0 | 0.16 | 0 | 1.24 | 1.97 |
| | Formulation 1 | 10 d | 0.93 | 0 | 0 | 0.08 | 1.03 | 1.39 |
| | Formulation 3 | 10 d | 0.91 | 0 | 0 | 0.06 | 0.98 | 1.27 |
| 40 °C RH25% | Formulation 5 | 5 d | 0.72 | 0 | 0.19 | 0 | 0.94 | 1.67 |
| | | 10 d | 0.83 | 0 | 0.34 | 0 | 1.20 | 1.93 |
| | Formulation 1 | 5 d | 0.49 | 0 | 0.08 | 0 | 0.57 | 0.93 |
| | | 10 d | 0.64 | 0 | 0.24 | 0 | 0.88 | 1.24 |
| | Formulation 3 | 5 d | 0.45 | 0 | 0.08 | 0 | 0.53 | 0.82 |
| | | 10 d | 0.69 | 0 | 0.29 | 0 | 0.98 | 1.27 |

As can be seen from the results in Table 6-3, under the condition of 40 °C, the stability of formulations 1 and 3 was superior to that of formulation 5, indicating that the stability of the pilocarpine phenylacetic acid series compound formulations of the present disclosure was superior to that of the pilocarpine hydrochloride formulation.

### Test Example 7

### Determination of Salt-Forming Ratios of Pilocarpine Salt Formulations

The determination of the salt-forming ratios of the pilocarpine salt formulations was performed using high-performance liquid chromatography with the following conditions: chromatographic column: C18 column; sample injection volume: 10 µL; mobile phase: K₂HPO₄ (adjusted to pH 6.50 ± 0.1 with phosphoric acid)-methanol-acetonitrile (63:2:35); flow rate: 0.7 mL/min; column temperature: 30 °C; detection wavelength: 215 nm; gradient elution determination was performed, and the salt-forming ratios were calculated according to the peak areas of free acid radicals and pilocarpine.

A specific amount of compound 1, boric acid, sodium citrate and sodium chloride was taken to prepare solutions with a concentration of 18.3 mg/mL (10.6 mg/mL based on pilocarpine) for compound 1, 10 mg/mL for boric acid, 0.15 mg/mL for sodium citrate and 1 mg/mL for sodium chloride, and then an appropriate amount of sodium hydroxide/hydrochloric acid was taken and added to each solution (for pH adjustment), thus preparing compound 1 formulation solutions with different pH values. The samples were separately injected to determine the salt-forming ratios of different compound 1 formulations. Meanwhile, the salt-forming ratios of the compound 1 formulation solutions with different pH values under different conditions were determined using the above stability examination methods. The results are shown in Table 7-1 below.

**Table 7-1:**

| Compound 1 formulations with different pH values | | Salt-forming ratio | | | | | | |
|---|---|---|---|---|---|---|---|---|
| No. | pH | 0d | Light irradiation/5 d | Light irradiation/10 d | 40 °C/5 d | 40 °C/10 d | 60 °C/5 d | 60 °C/10 d |
| 1 | 3.6 | 1.19 | 1.06 | 1.06 | 1.08 | 1.09 | 1.15 | 1.24 |
| 2 | 4.1 | 1.03 | 1.04 | 1.04 | 1.06 | 1.06 | 1.09 | 1.10 |
| 3 | 4.7 | 1.03 | 1.03 | 1.03 | 1.04 | 1.02 | 1.02 | 1.00 |
| 4 | 4.8 | 1.02 | 1.03 | 1.02 | 1.04 | 1.02 | 0.99 | 0.97 |
| 5 | 5.1 | 1.02 | 1.03 | 1.02 | 1.03 | 1.01 | 0.97 | 0.92 |
| 6 | 5.6 | 1.02 | 1.02 | 1.01 | 1.01 | 0.98 | 0.91 | 0.85 |

A specific amount of compound 2, boric acid, sodium citrate and sodium chloride was taken to prepare solutions with a concentration of 18.3 mg/mL (10.6 mg/mL based on pilocarpine) for compound 2, 10 mg/mL for boric acid, 0.15 mg/mL for sodium citrate and 1 mg/mL for sodium chloride, and then an appropriate amount of sodium hydroxide/hydrochloric acid was taken and added to each solution (for pH adjustment), thus preparing compound 2 formulation solutions with different pH values. The samples were separately injected to determine the salt-forming ratios of different compound 2 formulations. Meanwhile, the salt-forming ratios of the compound 2 formulation solutions with different pH values under different conditions were determined using the above stability examination methods. The results are shown in Table 7-2 below.

**Table 7-2:**

| Compound 2 formulations with different pH values | | Salt-forming ratio | | | | | | |
|---|---|---|---|---|---|---|---|---|
| No. | pH | 0 d | Light irradiation/5 d | Light irradiation/10 d | 40 °C/5 d | 40 °C/10 d | 60 °C/5 d | 60 °C/10 d |
| 1 | 4.3 | 1.25 | 1.12 | 1.13 | 1.14 | 1.15 | 1.17 | 1.23 |
| 2 | 4.5 | 1.00 | 1.01 | 1.01 | 1.02 | 1.01 | 1.02 | 1.01 |
| 3 | 4.8 | 1.00 | 1.01 | 1.01 | 1.01 | 1.00 | 0.98 | 0.96 |
| 4 | 5.0 | 1.00 | 1.00 | 1.00 | 1.00 | 0.99 | 0.95 | 0.92 |
| 5 | 5.5 | 0.99 | 1.00 | 1.00 | 0.98 | 0.97 | 0.90 | 0.85 |

As can be seen from the above table, under different concentrations and different conditions, the salt-forming ratios of the compound 1 and compound 2 formulations were substantially stable, demonstrating efficacy stability.

The embodiments of the present disclosure have been described above. However, the present disclosure is not limited to the embodiments described above. Any modification, equivalent replacement, improvement, and the like made without departing from the spirit and principle of the present disclosure shall fall within the protection scope of the present disclosure.

## Claims

1. A compound represented by formula (A) or a pharmaceutically acceptable hydrate or solvate thereof: 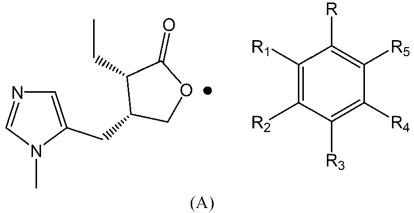
wherein R₁, R₂, R₃, R₄, and R₅ are identical or different, and are each independently selected from hydrogen, C₁₋₆ alkyl, halogen, cyano, and -NR'R", wherein R' and R" are identical or different, and are each independently selected from H, C₁₋₆ alkyl, C₃₋₁₀ cycloalkyl, C₆₋₁₄ aryl, 5- to 14-membered heteroaryl, and 3- to 10-membered heterocyclyl, wherein the C₁₋₆ alkyl, C₃₋₁₀ cycloalkyl, C₆₋₁₄ aryl, 5- to 14-membered heteroaryl and 3- to 10-membered heterocyclyl are optionally substituted with one, two or more of the following groups: oxo, thio, halogen, cyano, -NR'R", -COOH, hydroxyl, C₁₋₆ alkyl, C₁₋₆ alkoxy, C₁₋₆ alkylthio, C₃₋₁₀ cycloalkyl, C₆₋₁₄ aryl, and 5- to 14-membered heteroaryl; and
R is selected from COOH or 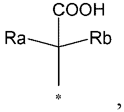 wherein Ra and Rb are identical or different, and are each independently selected from hydrogen, C₁₋₆ alkyl, halogen, cyano, C₃₋₁₀ cycloalkyl, C₆₋₁₄ aryl, 5- to 14-membered heteroaryl, and 3- to 10-membered heterocyclyl.

2. The compound as claimed in claim 1, wherein the compound represented by formula (A) is selected from a compound represented by formula I-1 or formula I-2 shown below:

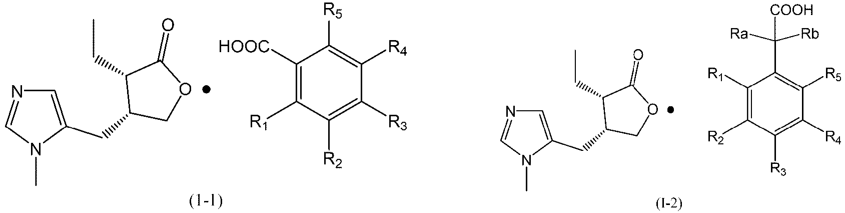

3. The compound as claimed in claim 1 or 2, wherein in formula I-1, R₁, R₂, R₃, R₄ and R₅ are identical or different, and are each independently selected from hydrogen, C₁₋₆ alkyl, halogen, cyano, and amino; or
in formula I-2, R₁, R₂, R₃, R₄, and R₅ are identical or different, and are each independently selected from hydrogen, C₁₋₆ alkyl, halogen, and -COOR', wherein R' is independently selected from H and C₁₋₆ alkyl.

4. The compound as claimed in claim 3, wherein in formula I-1, at least three of R₁, R₂, R₃, R₄, and R₅ are H; or
in formula I-2, R₁, R₂, R₃, R₄, and R₅ are identical or different, and are each independently selected from hydrogen, methyl, ethyl, isopropyl, chlorine, fluorine, and bromine; or
in formula I-2, Ra and Rb are identical or different and are each independently selected from hydrogen, C₁₋₆ alkyl, halogen, and cyano.

5. The compound as claimed in claim 1, wherein the compound of formula I-1 is selected from the following:
| **Compound#** | **Structure** |
|---|---|
| JQ-1 | 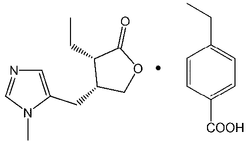 |
| JQ-2 | 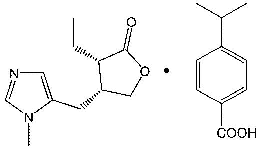 |
| JQ-3 | 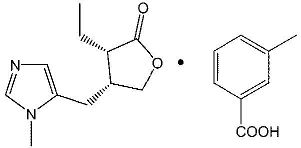 |
| JQ-5 | 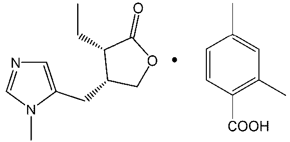 |
| JQ-6 | 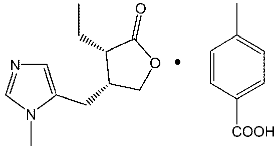 |
| JQ-8 | 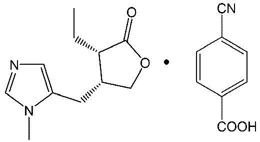 |
| JQ-9 | 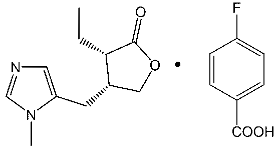 |
| JQ-10 | 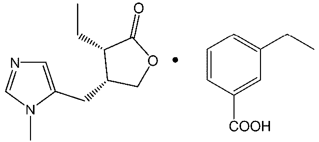 |
the compound of formula I-2 is selected from the following:
| No. | Compound |
|---|---|
| 1 | 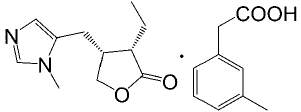 |
| 2 | 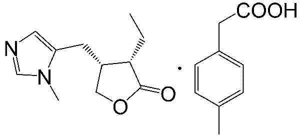 |
| 3 | 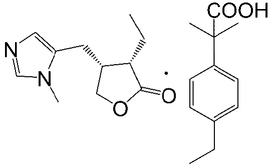 |
| 4 | 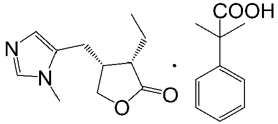 |
| 5 | 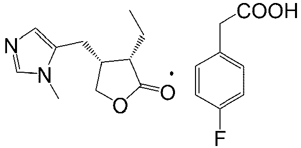 |
| 6 | 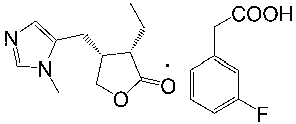 |
| 7 | 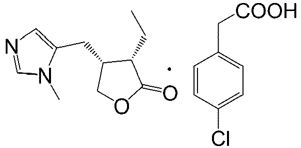 |
| 8 | 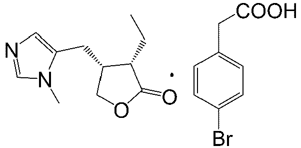 |
| 9 | 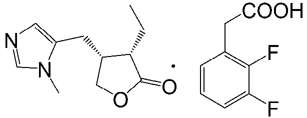 |
| 10 | 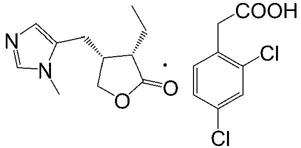 |
| 11 | 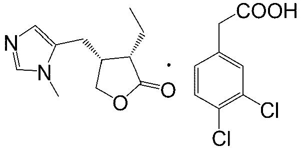 |
| 12 | 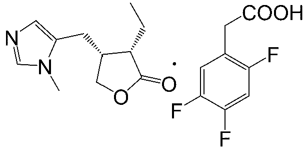 |
| 13 | 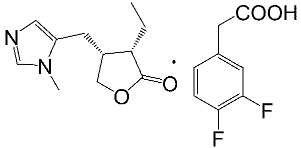 |
| 14 | 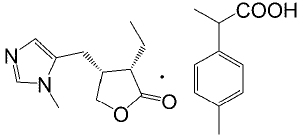 |
| 15 | 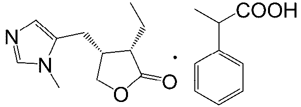 |

6. A preparation method for the compound represented by formula (A) as claimed in any one of claims 1-5, comprising: reacting pilocarpine with a compound of formula (A-1) to give the compound represented by formula (A): 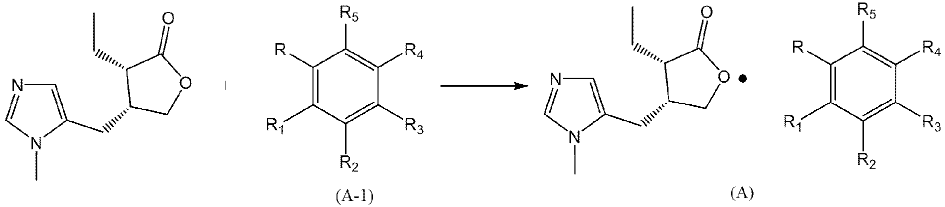 wherein R, R₁, R₂, R₃, R₄, and R₅ are defined as in any one of claims 1-5.

7. A pharmaceutical composition, comprising at least one of the compounds represented by formula (A) or the pharmaceutically acceptable hydrate or solvate thereof as claimed in any one of claims 1-5.

8. The pharmaceutical composition as claimed in claim 7, wherein in the pharmaceutical composition, the mass concentration of the compound represented by formula (A) is 0.3%-8%.

9. Use of the compound represented by formula (A) as claimed in any one of claims 1-5 or the pharmaceutical composition as claimed in claim 7 or 8 in the manufacture of a medicament for the amelioration or treatment of an ocular disease or disorder, or a medicament for miosis.

10. The use as claimed in claim 9, wherein the ocular disease or disorder is at least one of presbyopia, cataract, glaucoma, dry eye syndrome, diabetic retinopathy, macular degeneration, optic neuritis, and retinitis pigmentosa;
preferably, the ocular disease or disorder is presbyopia.
